# EUROPEAN PATENT SPECIFICATION

(11) **EP 4 203 925 B1**
(45) Date of publication and mention of the grant of the patent: **16.09.2026**
(21) Application number: 21766227.9
(22) Date of filing: 31.08.2021
(51) Int. Cl.: A61K 31/352, A61P 35/00, A61K 31/00

(54) **CANNABINOID COMBINATIONS AND THEIR USE IN THE TREATMENT OF CANCER**
CANNABINOID-KOMBINATIONEN UND DEREN VERWENDUNG BEI DER BEHANDLUNG VON KREBS
ASSOCIATIONS DE CANNABINOÏDES ET LEUR UTILISATION DANS LE TRAITEMENT DU CANCER

(30) Priority: 31.08.2020 ZA 202005408
(43) Date of publication of application: 05.07.2023
(73) Proprietor: Nelson Mandela University, 6001 Port Elizabeth (ZA)
(72) Inventor: FROST, Carminita Lara, 6001 Port Elizabeth (ZA); BEUKES, Natasha, 6001 Port Elizabeth (ZA); SCHOEMAN, Recardia Laken Simoney, 6001 Port Elizabeth (ZA); LEVENDAL, Ruby-Ann, 6001 Port Elizabeth (ZA)
(74) Representative: Potter Clarkson
(86) International application number: PCT/IB2021/057931
(87) International publication number: WO 2022/043961

(56) References cited:
- WO-A1-2020/121312
- GB-A- 2 478 595
- GB-A- 2 495 841
- GB-A- 2 560 019
- SCHOEMAN RECARDIA ET AL: "Cannabinoid Combination Induces Cytoplasmic Vacuolation in MCF-7 Breast Cancer Cells", MOLECULES, vol. 25, no. 20, 14 October 2020 (2020-10-14), pages 4682, XP055862130, DOI: 10.3390/molecules25204682
- DARIS BARBARA ET AL: "Cannabinoids in cancer treatment: Therapeutic potential and legislation", BOSNIAN JOURNAL OF BASIC MEDICAL SCIENCES, vol. 19, no. 1, 12 February 2019 (2019-02-12), Sarajevo , Bosnia, pages 14 - 23, XP055863036, ISSN: 1512-8601, Retrieved from the Internet <URL:http://www.bjbms.org/ojs/index.php/bjbms/article/download/3532/1121> DOI: 10.17305/bjbms.2018.3532

## Description

### INTRODUCTION

This invention relates to a combination of cannabinoids for use in the treatment of cancer, and a pharmaceutical composition comprising the combination. In particular, but not exclusively, this invention relates to a synergistic combination of cannabinoids, a pharmaceutical composition comprising the synergistic combination, and the use of the combination or pharmaceutical composition in the treatment of cancer, in particular breast cancer.

### BACKGROUND

*Cannabis sativa* L. has been used for centuries in the treatment of various ailments. One particular group of compounds produced by this plant, are the C₂₁ terpenophenolics (referred to as cannabinoids), are well known for their vast range of bioactivity. There are several types of cannabinoids: endocannabinoids, synthetically synthesised cannabinoids and phytocannabinoids, which specifically refer to the cannabinoids obtained from *C. sativa.* The most abundant and psychoactive phytocannabinoid is Δ⁹-tetrahydrocannabinol. Other well-known phytocannabinoids include: cannabigerol (CBG), cannabinol (CBN), cannabidiol (CBD), cannabichromeme (CBC), cannabicyclol and (CBL), amongst others.

Phytocannabinoids have been found to mimic endogenous cannabinoids by activating cannabinoid receptors, which, depending on the cell type, allows phytocannabinoids to modulate cell proliferation, differentiation and cell death. This ability, gained traction in the cancer field, since the activation of cannabinoid receptor(s) can be exploited to influence several hallmarks of tumour progression.

The anti-cancer effects of phytocannabinoids have been observed in several cancer types, including gliomas, and carcinomas of the skin, liver, colon, prostate and breast. Breast cancer is particularly difficult to treat due to its heterogeneity. Breast cancer cells are heterogenous and are mainly classified by the expression of the hormone receptors (oestrogen and progesterone) and the epidermal growth factor receptor 2 (HER2). Breast cancer cells that lack these receptors are referred to as triple negative breast cancer (TNBC). TNBC are aggressive and notoriously difficult to treat due to their lack of targets.

Studies have shown that phytocannabinoids are effective against various breast cancer subtypes by inducing cell cycle arrest and cell death via pathways such as apoptosis and autophagy. Several preclinical studies have demonstrated the anti-tumourigenic effects of cannabinoids against breast cancer cells; however, these studies mainly focused on single cannabinoid formulations of either CBD or THC. CBD and THC have been documented to be anti-proliferative, pro-apoptotic and anti-migratory in several in vitro and in vivo breast cancer models.

Furthermore, although conventional chemotherapeutic agents exist, patients often experience side-effects that affects their quality of life. In addition, cancers often acquire resistance mechanisms to evade cell death pathways, in which the chemotherapeutic agent may become obsolete. Several studies have evaluated the use of drug combinations to overcome these chemotherapy-associated problems. Some advantages include: the reduction of the required dose, minimal potential to induce toxicity in the host, reduction of the cost associated with therapy and minimal risk of developing drug resistance have been associated with the various studies.

Consequently, the inventors of the present invention investigated the use of cannabinoid combinations to amplifying its therapeutic efficacy by simultaneously activating multiple anti-cancer mechanisms in breast cancer cell lines. Surprisingly, the inventors found that combinations of cannabinoids in certain ratio ranges are synergistic against MDA-MB-231 and MCF-7 cancer cell lines.

WO 2020/121312 A1 discusses compositions containing cannabinoids and their use in methods for the treatment of cutaneous T cell lymphoma.

### SUMMARY OF THE INVENTION

The invention is as described in the appended claims.

According to a first aspect to the present invention there is provided a combination comprising Δ⁹-tetrahydrocannabinol (THC), cannabigerol (CBG), cannabinol (CBN), and cannabidiol (CBD) in a ratio of 3.7 parts THC, 1.0 parts CBG, 1.9 parts CBN, and 4.8 parts CBD, or wherein the combination comprises 5.5 parts THC, 1.0 parts CBG, 1.3 parts CBN, and 4.8 parts CBD, for use in a method of treating cancer.

Also described herein, that does not fall within the literal scope of the claims, is a cannabinoid combination, the combination comprising Δ⁹-tetrahydrocannabinol (THC), cannabigerol (CBG), cannabinol (CBN), and cannabidiol (CBD) in a ratio of about 1.0 - 6.0 parts THC, about 0.5 - 3.5 parts CBG, about 0.5 - 3.0 parts CBN, and about 0.5 - 6.0 parts CBD, for use in a method of treating cancer.

In an example described herein, that does not fall within the literal scope of the claims, the combination comprises Δ⁹-tetrahydrocannabinol (THC), cannabigerol (CBG), cannabinol (CBN), and cannabidiol (CBD) in a ratio of about 2.0 - 5.5 parts THC, about 1.0 - 2.5 parts CBG, about 1.0 - 2.0 parts CBN, and about 1.0 - 5.0 parts CBD.

In an example described herein, that does not fall within the literal scope of the claims, the combination comprises about 2.6 parts THC, about 2.4 parts CBG, about 1.3 parts CBN, and about 1.0 parts CBD.

In an example described herein, that does not fall within the literal scope of the claims, the combination comprises about 2.0 parts THC, about 1.2 parts CBG, about 1.0 parts CBN, and about 1.1 parts CBD.

In an example described herein, that does not fall within the literal scope of the claims, the combination comprises about 2.4 parts THC, about 1.0 parts CBG, about 2.8 parts CBN, and about 4.8 parts CBD.

In another embodiment, the combination comprises about 3.7 parts THC, about 1.0 parts CBG, about 1.9 parts CBN, and about 4.8 parts CBD (C6).

In another embodiment, the combination comprises about 5.5 parts THC, about 1.0 parts CBG, about 1.3 parts CBN, and about 4.8 parts CBD (B6).

In an example described herein, that does not fall within the literal scope of the claims, the cannabinoids are present at a concentration of about 10 µM - 30 µM THC, about 3 µM - 30 µM CBG, about 6 µM - 20 µM CBN, and 10 µM - 40 µM CBD.

In an example described herein, that does not fall within the literal scope of the claims, the cannabinoids are present at a concentration of about 28.89 µM THC, about 26.67 µM CBG, about 14.82 µM CBN, and 11.26 µM CBD.

In an example described herein, that does not fall within the literal scope of the claims, the cannabinoids are present at a concentration of about 28.89 µM THC, about 17.78 µM CBG, about 14.82 µM CBN, and 16.89 µM CBD.

In an example described herein, that does not fall within the literal scope of the claims, the cannabinoids are present at a concentration of about 28.89 µM THC, about 11.85 µM CBG, about 14.82 µM CBN, and 25.33 µM CBD.

In an example described herein, that does not fall within the literal scope of the claims, the cannabinoids are present at a concentration of about 28.89 µM THC, about 7.90 µM CBG, about 14.82 µM CBN, and 38.00 µM CBD.

In an example described herein, that does not fall within the literal scope of the claims, the cannabinoids are present at a concentration of about 12.68 µM THC, about 3.46 µM CBG, about 6.50 µM CBN, and 16.68 µM CBD.

In one embodiment, the cancer is breast cancer.

In a preferred embodiment, the combination is a synergistic combination.

According to a second aspect to the present invention there is provided a pharmaceutical composition comprising 3.7 parts Δ⁹-tetrahydrocannabinol (THC), 1.0 parts cannabigerol (CBG), 1.9 parts cannabinol (CBN), and 4.8 parts cannabidiol (CBD), or 5.5 parts THC, 1.0 parts CBG, 1.3 parts CBN, and 4.8 parts CBD, and one or more pharmaceutically acceptable excipients.

Also disclosed herein is a pharmaceutical composition comprising about 1.0 - 6.0 parts Δ⁹-tetrahydrocannabinol (THC), about 0.5 - 3.5 parts cannabigerol (CBG), about 0.5 - 3.0 parts cannabinol (CBN), and about 0.5 - 6.0 parts cannabidiol (CBD) and one or more pharmaceutically acceptable excipients.

Also disclosed herein is a pharmaceutical composition comprising about 2.0 - 5.5 parts Δ⁹-tetrahydrocannabinol (THC), about 0.5 - 3.5 parts cannabigerol (CBG), about 0.5 - 3.0 parts cannabinol (CBN), and about 0.5 - 6.0 parts cannabidiol (CBD) for use in a method of treating cancer.

Preferably, the cancer is breast cancer.

According to another aspect to the present invention there is provided a method of treating cancer, the method comprising administering to a subject in need thereof a therapeutically effective amount of a cannabinoid combination, the combination comprising a synergistic combination of Δ9-tetrahydrocannabinol (THC), cannabigerol (CBG), cannabinol (CBN), and cannabidiol (CBD).

The references to the methods of treatment by therapy in this description are to be interpreted as references to combinations and pharmaceutical compositions of the present invention for use in those methods.

In a preferred embodiment, the combination comprises Δ⁹-tetrahydrocannabinol (THC), cannabigerol (CBG), cannabinol (CBN), and cannabidiol (CBD) in a ratio of about 1.0 - 6.0 parts THC, about 0.5 - 3.5 parts CBG, about 0.5 - 3.0 parts CBN, and about 0.5 - 6.0 parts CBD.

### BRIEF DESCRIPTION OF THE DRAWINGS

The invention will now be described in more detail with reference to the following non-limiting embodiments and figures in which:
- **Figure 1**: shows Dose response curves of individual cannabinoids in the MCF-7 and MDA-MB-231 cell lines;
- **Figure 2**: shows median effect plots of individual cannabinoids in the MDA-MB-231 and MCF-7 cell lines;
- **Figure 3**: shows dose response curves consisting of the various combinations of two cannabinoids, determined against breast cancer cell lines;
- **Figure 4**: shows median effect plots for the various combinations of two cannabinoids in the MDA-MB-231 cell line;
- **Figure 5**: shows median effect plots for combinations of two cannabinoids in MCF-7 breast cancer cells;
- **Figure 6**: shows an illustration of the combination of four cannabinoids in a 96-well cell culture plate for treatment of the breast cancer cell lines, MDA-MB-231 and MCF-7;
- **Figure 7**: shows Checkerboard assay consisting of four cannabinoid combinations at various ratios with cannabinoid ratios represented in the order THC:CBG:CBN:CBD;
- **Figure 8**: shows growth inhibition obtained using variations of the four cannabinoid combinations in breast cancer cell lines;
- **Figure 9**: shows combination index values obtained for the various combinations of the four cannabinoids in breast cancer cell lines;
- **Figure 10**: shows dose response curves of selected, four-cannabinoid combinations in MDA-MB-231 cells;
- **Figure 11**: shows dose response curves of selected, four-cannabinoid combinations in MCF-7 cells;
- **Figure 12**: shows median effect plots of selected four cannabinoid ratios that displayed synergistic interactions in MDA-MB-231 breast cancer cells;
- **Figure 13**: shows median effect plots obtained with the MCF-7 cell line, after treatment with selected four-cannabinoid combinations;
- **Figure 14**: shows the screening of synergistic ratios in the non-cancerous MCF-10A and white blood cells;
- **Figure 15**: shows typical images obtained of MCF-7 cells with phase contrast (40x magnification). Cells were treated with A) DMSO vehicle control and B) the synergistic cannabinoid combination **C6** (46 µM) for 48 hours, where after images were taken using the 20x objective of the Zeiss Axio fluorescence microscope;
- **Figure 16**: shows representative images of MCF-7 cells stained with ER Tracker^{™} and CytoPainter^{™}. After treatment with **C6** (46 µM). Treated cells were stained with A) ER Tracker^{™} which stains the endoplasmic reticulum and B) CytoPainter^{™} which stains the mitochondria. Images were acquired using the ImageXpress Micro XLS Widefield High-Content Analysis System (10x magnification). C) Cells stained with Cytopainter^{™} and imaged using the Zeiss LSM510 Meta laser scanning confocal microscope;
- **Figure 17**: shows western blot analysis of GRP78 protein levels in MCF-7 cells treated with **C6** (46 µM). A) Relative protein expression levels of GRP78 in MCF-7 cells treated with **C6**. Protein levels were normalised to total protein and expressed as a fold change relative to the DMSO vehicle control. B) Representative membranes showing band intensity of the GRP78 levels. Error bars represent SEM and *p<0.05 relative to DMSO vehicle control using one way ANOVA (n=3);
- **Figure 18**: shows western blot analysis of p-ERK 1 and p-ERK 2 in MCF-7 cells. A) Relative protein expression levels of phosphorylated p44/p42 MAPK (ERK1/2) of MCF-7 cells treated with **C6** (46 µM). Protein levels were normalised to total protein and expressed as a fold change relative to the DMSO vehicle control. B) Representative membrane showing band intensity of the p-ERK 1 and p-ERK 2 levels. Error bars represent SEM, *p<0.05 relative to DMSO vehicle control using one way ANOVA (n=3);

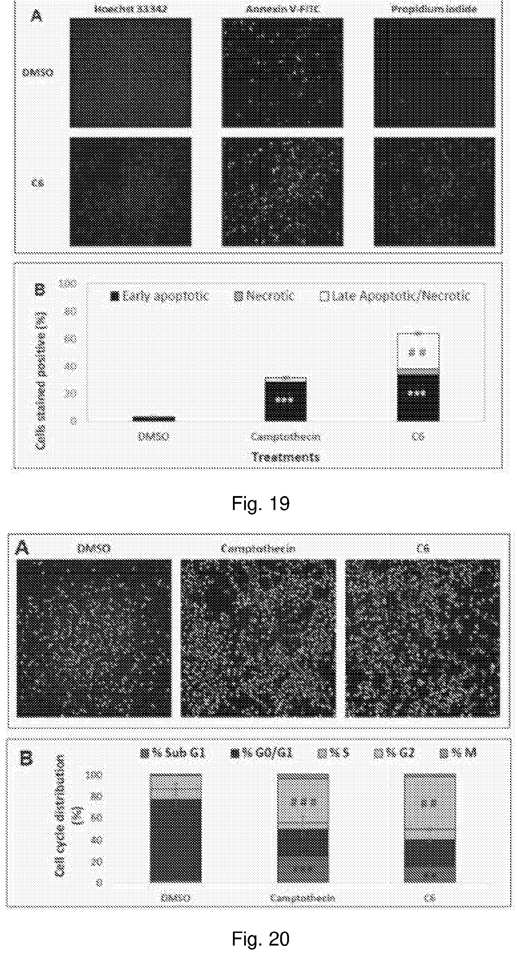

- **Figure 19**: shows annexin V-FITC and propidium iodide staining of MCF-7 cells. Cells were treated with **C6** at 70 µM. A) Representative images of cells stained with Hoechst 33342 to determine the cell number per images acquired, cells stained with annexin V-FITC to determine the percentage cells stained positive for phosphatidylserine translocation and cells stained with propidium iodide to establish the plasma membrane integrity of the cell. B) Quantitative analysis of the cell population undergoing apoptosis or necrosis. Positive staining with Annexin V only, is indicative of early apoptosis, positive staining with propidium iodide only is indicative of necrosis, while positive staining with both dyes indicates either necrosis or late apoptosis. Camptothecin (29 µM) was used as a positive control. Images were acquired using the ImageXpress Micro XLS Widefield High-Content Analysis System. Data represents average of the percentage cells stained positive per image, normalised to the cell number per images acquired. Error bars represent SEM; * p<0.05 and ** p<0.01, *p<0.001 relative to DMSO of % early apoptotic cells; ^{#} p<0.05 and ^{##} p<0.01,^{###} p<0.001 relative to DMSO of % late apoptotic/necrotic cells using one way ANOVA with post hoc Tukey test (n=3); and
- **Figure 20**: shows cell cycle analysis of MCF-7 cells after treatment with **C6** (46 µM). A) Representative images acquired with colour overlays, where each colour represents the phase of the cell cycle assigned to the specific cell, based on the intensity of the Hoechst 33342 staining. B) Quantitative analysis of the percentage of the cell population in each phase of the cell cycle. Camptothecin (5.74 µM) was used as a positive control for arrest in the G2 phase. Error bars represent SEM; **p<0.01, ***p<0.001 relative to DMSO vehicle control of % Sub G1; ^{#}p<0.05, ^{# #}p<0.01, ^{# # #}p<0.001 relative to DMSO vehicle control of %G2 using one way ANOVA with post hoc Tukey test (n=3).

### DETAILED DESCRIPTION OF A PREFERRED EMBODIMENT

The present invention will now be described more fully hereinafter with reference to the accompanying figures, in which some of the non-limiting embodiments of the invention are shown.

Although specific terms are employed herein, they are used in a generic and descriptive sense only and not for purposes of limitation.

As used herein, throughout this specification and in the claims which follow, the singular forms "a", "an" and "the" include the plural form, unless the context clearly indicates otherwise.

The terminology and phraseology used herein is for the purpose of description and should not be regarded as limiting. The use of the terms "comprising", "containing", "having", "including", and variations thereof used herein, are meant to encompass the items listed thereafter, and equivalents thereof as well as additional items.

The invention provides broadly for a combination of cannabinoids for use in the treatment of cancer, and a pharmaceutical composition comprising the combination. In one embodiment the cancer is breast cancer. In one embodiment the invention provides for a synergistic combination of cannabinoids, a pharmaceutical composition comprising the synergistic combination, and the use of the combination or pharmaceutical composition in the treatment of cancer, which may be breast cancer.

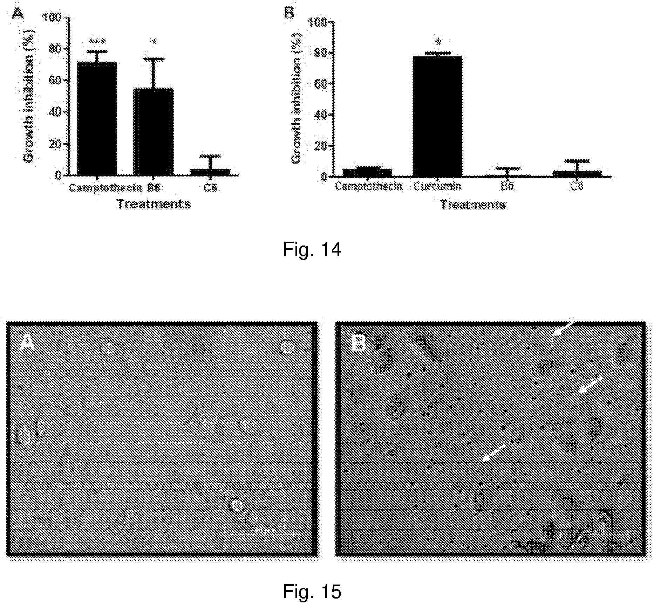

Various cannabinoid combinations were evaluated for potential synergistic interactions against the MDA-MB-231 and MCF-7 breast cancer cell lines. Camptothecin was used as the positive control for anti-proliferative activity. Dose response curves of each cannabinoid combination was constructed and subsequently linearised to create median-effect plots. The linear equations obtained from the median-effect plots were used to determine the median effect parameters (*e.g*. slope, linear correlation coefficient and potency). These parameters were used to quantitate the interactions between the cannabinoids using the Chou-Talalay method. The most promising synergistic combinations were selected for further studies, which included screening in non-cancerous cells, such as the normal breast cell line, MCF-10A and cytotoxicity screening in isolated white blood cells (Figure 14). The promising synergistic combinations were further evaluated for reductions in the required dose using the dose reduction index across the cell line in which the synergism was established (Table 5).

The invention provides for a cannabinoid combination, the combination comprising Δ⁹-tetrahydrocannabinol (THC), cannabigerol (CBG), cannabinol (CBN), and cannabidiol (CBD) in a ratio of 3.7 parts THC, 1.0 parts CBG, 1.9 parts CBN, and 4.8 parts CBD, or wherein the combination comprises 5.5 parts THC, 1.0 parts CBG, 1.3 parts CBN, and 4.8 parts CBD, for use in a method of treating cancer. Preferably, the combination is a synergistic combination. The invention further provides for a pharmaceutical composition comprising the composition, and to a method of treatment, the method comprising administering to a patient in need thereof a therapeutic amount of a combination comprising Δ⁹-tetrahydrocannabinol (THC), cannabigerol (CBG), cannabinol (CBN), and cannabidiol (CBD).

An example disclosed herein, that does not fall within the literal scope of the claims, the combination for use comprises Δ⁹-tetrahydrocannabinol (THC), cannabigerol (CBG), cannabinol (CBN), and cannabidiol (CBD) in a ratio of about 1.0 - 6.0 parts THC, about 0.5 - 3.5 parts CBG, about 0.5 - 3.0 parts CBN, and about 0.5 - 6.0 parts CBD. Preferably the combination comprises Δ⁹-tetrahydrocannabinol (THC), cannabigerol (CBG), cannabinol (CBN), and cannabidiol (CBD) in a ratio of about 2.0 - 5.5 parts THC, about 1.0 - 2.5 parts CBG, about 1.0 - 2.0 parts CBN, and about 1.0 - 5.0 parts CBD. These ranges are provided as illustrative examples of the invention only, it being understood that the ranges specifically include subranges falling within the specified ranges, for example, 1.1, 1.2, 1.3, 1.4, 1.5 and increments of 0.1 up to the upper range limit of 6.0 for THC. The same applies for the ranges specified for CBG, CBN, and CBD.

### EXPERIMENTAL METHODS

All reagents and chemicals used were of an analytical grade.

All experiments were performed in triplicate (n=3). The values obtained were expressed as mean ± standard error unless otherwise stated. Analysis of variance (ANOVA) with *post hoc* Tukey test was used to analyse the significance between various treatments relative to vehicle controls. Statistics was calculated using GraphPad Prism^{®} 5 version 5.01. Statistical significance was set at p<0,05.

### Cell Maintenance

The human breast cancer cell line, MDA-MB-231, was cultured in Leibovitz's L-15 medium (Sigma-Aldrich) supplemented with 10% (v/v) foetal bovine serum (FBS) and the MCF-7 cell line was cultured in Dulbecco's Modified Eagle's Medium (DMEM) high glucose supplemented with 25mM (4-(2-hydroxyethyl)-1-piperazineethanesulfonic acid (HEPES) and 10% (v/v) FBS (Biowest). The non-cancerous breast cell line, MCF-10A, was cultured in DMEM/F12 (Biowest^{®}) supplemented with 5% (v/v) donor horse serum, 3.3 nM epidermal growth factor (EGF) (Gibco^{®}, Life Technologies), 1.74 µM insulin (Sigma-Aldrich) and 1.38 µM hydrocortisone (Sigma-Aldrich). All cell lines were obtained from the American Type Culture Collection (Manassas, VA). White blood cells isolated from healthy human volunteers were cultured in Roswell Park Memorial Institute-1640 (RPMI-1640) (with 25mM HEPES; L-glutamine) supplemented with 10% (v/v) FBS. For sub-culturing, spent media was aspirated, the cells washed with PBS-EDTA (pH 7.4) followed by the addition of 0.25% (v/v) Trypsin-EDTA. Cells were incubated at 37 °C until cells were detached. After cell detachment, fresh culture media was added to the cells and the cells split in a sub-cultivation ratio of 1:2 to 1:4. Cultures were maintained at 37 °C in humidified atmosphere with a 5% CO₂ concentration, except for the MDA-MB-231 cells which were incubated in an airtight container to limit the CO₂ present in the culture environment.

### Treatment

Cells were trypsinised and seeded at a cell density of 1 x 10⁴ cells per well (100 µl) in a 96-well plate and allowed to attach overnight. The media was replaced with 100µl of fresh media supplemented with the appropriate cannabinoid treatment. The cells were exposed to the treatment(s) for 48 hours at 37 °C under the same growth conditions described above. Cannabinoid standards supplied in methanol were evaporated under nitrogen gas and reconstituted in DMSO to prepare stock solutions. Working solutions were prepared in the appropriate cell culture media for the respective cell line and included appropriate DMSO vehicle controls. All dose response curves were constructed using GraphPad Prism^{®} 5 version 5.01 and data summarised the concentrations required to inhibit the growth of the cell population by 50% (IC₅₀,), 75% (IC₇₅) and 90% (IC₉₀). Treatments were performed as set out below.

### Dose response curves: Single cannabinoids

Breast cancer cell lines, MDA-MB-231 and MCF-7 were treated with a range of cannabinoid concentrations (16, 24, 32, 48 and 64 µM) to prepare dose response curves for the individual cannabinoids (THC, CBG, CBN and CBD). The inhibitory concentrations (IC values) are summarised in Table 1.

### Dose response curves: Two cannabinoid combination

Dose response curves using the four cannabinoids were prepared using a range of permutations of two-cannabinoid combinations, *i.e.* THC:CBG, THC:CBN, THC:CBD, CBG:CBN and finally CBD:CBN.

Dose response curves were constructed with both cannabinoids at double their respective IC₅₀ concentrations. Ideally, a dose response curve should contain data points along the shape of the curve, including regions of the maximal and minimal drug effects (top and bottom plateau) and the exponential region of the sigmoidal curve. Since the two-fold drug dilutions resulted in data points only fitted along the maximal and minimal regions, the dilution fold was decreased to ensure that experimental data points were scattered along the regions of minimal and maximal drug effects, as well as the exponentially increasing region of the sigmoidal curve. Cannabinoid solutions were therefore serially diluted 1.5-fold in the 96-well culture plate by adding 200 µl of the cannabinoid working solution to 100 µl of culture media. The solution was then mixed by pipetting and the dilution repeated to create a six-point dose response curve. Cannabinoid interactions were then quantified using the Chou-Talalay method (Chou, 2010) with the CompuSyn software. An array of four cannabinoid ratios were screened for potential synergistic interactions.

### Four cannabinoid combination: checkerboard assay

Four cannabinoids were combined using the checkerboard assay (Kars *et al.,* 2006). The checkerboard assay consisted of four cannabinoids, each at six different concentrations. The maximum concentration of individual cannabinoids was double their respective IC₅₀ values, with ICso values as follows: THC (32 µM), CBG (30 µM), CBN (25 µM) and CBD (19 µM).

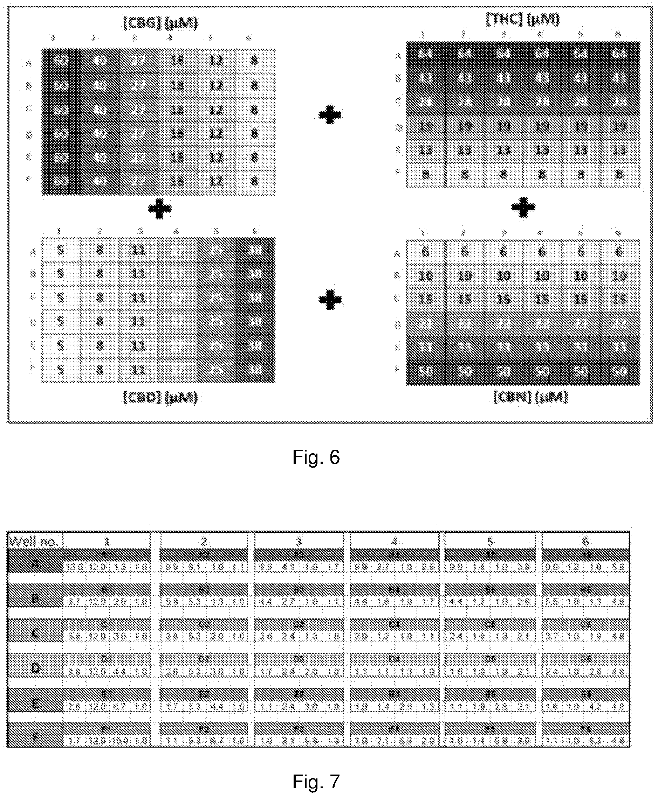

Four cannabinoids were added to a respective well with each cannabinoid at a different concentration. Each well consisted of 25 µl of each respective cannabinoid, adding up to a final volume of 100 µl per well, thereby creating a four-fold dilution factor. To ensure that respective cannabinoids had a maximum concentration of double its ICso value, the dilution factor was taken into account and stock concentrations of individual cannabinoids were prepared: THC (256 µM), CBG (240 µM), CBN (200 µM) and CBD (152 µM). The stock concentrations were subsequently diluted 1.5 fold in microcentrifuge tubes to create the six different concentrations and added to the cell culture plate following the layout illustrated (Figure 6). For example, from Figure 6 it can be seen that the cell culture well **B3** consisted of all four cannabinoids at final concentrations of: THC (43 µM), CBG (27 µM), CBN (10 µM) and CBD (11 µM). Since all four cannabinoids overlapped at different concentrations within the cell culture plate, the checkerboard assay created multiple cannabinoid ratios as a screening method for potential synergistic ratios. Drug interactions were evaluated using the Chou-Talalay method (Chou, 2010).

### MTT cell viability assay

### Breast cell lines

To evaluate the effects of the cannabinoid treatments on the breast cancer cell lines, MDA-MB-231 and MCF-7 and the non-cancerous breast cell line MCF-10A, spent media containing the treatment was replaced with 200µl fresh media supplemented with 0.5 mg/ml MTT, the cells were incubated for 2 hours at 37 °C. The excess MTT was removed and 200µl of DMSO was added to solubilise the formazan crystals and absorbance measured at 550nm using an EPOCH 2 microplate reader^{™} (BioTek^{®} Instruments Inc., USA). Cell density standard curves were used to normalise the data to cell number.

### White blood cells

MTT solution (5mg/ml, 20 µl) was added to existing media in the wells and the cells were incubated for 3 hours at 37 °C. After the 3 hours, the plate was centrifuged at 1000 x g for 10 minutes, the spent media removed and the formazan crystals solubilised in 100 µl DMSO. The plate was then shaken for 1 minute and the absorbance read at 550nm. Cell density standard curves were used to normalise the data to cell number.

### Evaluating cannabinoid-induced toxicity in non-tumorigenic cells

### Toxicity in non-tumorigenic breast cells

MCF-10A cells were seeded at 1.5 x 10⁴ cells/well and left to attach overnight. The spent media was aspirated and replaced with 100 µl fresh media containing the IC₉₀ of synergistic cannabinoid combinations. Cells were exposed to treatment for 48 hours, followed by the determination of cell viability using the MTT assay.

### Toxicity screening in white blood cells

Blood samples were obtained from healthy volunteers (n=3, 16 ml/person) in ethylenediaminetetraacetic acid (EDTA) vacutainers. The samples were centrifuged at 500 x g for 10 minutes, the plasma (top layer) removed and the buffy coat collected. The buffy coat was then centrifuged at 2000 x g for 10 minutes to remove residual plasma while retaining the buffy coat. Lysis buffer (0.16M NH₄Cl, 0.01 M KHCO₃, 6 mM EDTA; pH 7.3) was added to the buffy coat in a 9:1 ratio (lysis buffer:buffy coat) and the mixture vortexed for 10 seconds, followed by a 10 minute incubation at room temperature. The sample was then centrifuged at 2000 x g for 10 minutes and the pellet retained (containing white blood cells). The pellet was washed with lysis buffer, resuspended and centrifuged at 1000 x g for 5 minutes. The pellet was retained, and the washing step repeated. The isolated white blood cells were diluted in RPMI 1640 medium (with 25 mM HEPES and L-glutamine), supplemented with 10% (v/v) FBS. White blood cells were stained with trypan blue and counted using a haemocytometer. Cells were then seeded at 25 x 10⁴ cells/well (100 µl) in a 96-well cell culture plate and immediately exposed to the synergistic cannabinoid combinations at the 2 x IC₉₀ concentrations to account for the additional 100 µl treatment added to each well (two-fold dilution). Treatments were made up in RPMI 1640. The IC₉₀ was selected to determine how selective the promising synergistic cannabinoid treatments were to breast cancer cells, while displaying limited toxicity to the non-cancerous cells, especially at the high treatment concentrations. Cells were exposed to treatment for 24 hours (standardised lab protocol), followed by the determination of cell viability using the MTT assay.

### Determination of the dose reduction index

The objective of a synergistic combination is to decrease the required dose of a drug, while still retaining its efficacy. The dose reduction index (DRI) (Chou and Talalay, 1984) measured the fold by which the concentration of each drug in a synergistic combination can be decreased to induce "x" % inhibition, in comparison to the concentration required of each drug alone to induce the same "x" % inhibition. The dose reduction was calculated using the following equation, where a DRI > 1 is considered beneficial. $CI = {\sum }_{j = 1}^{n} \frac{{\left(D\right)}_{j}}{{\left({D}_{x}\right)}_{j}} = {\sum }_{j = 1}^{n} \frac{1}{{\left(DRI\right)}_{j}}$ *Therefore,* ${\left(DRI\right)}_{1} = \frac{{\left({D}_{x}\right)}_{1}}{{\left(D\right)}_{1}} , {\left(DRI\right)}_{2} = \frac{{\left({D}_{x}\right)}_{2}}{{\left(D\right)}_{2}} , … {\left(DRI\right)}_{n} = \frac{{\left({D}_{x}\right)}_{n}}{{\left(D\right)}_{n}}$ *Where* ${\left({D}_{x}\right)}_{n} = {\left({D}_{m}\right)}_{n} {\left[{f}_{a}/\left(1-{f}_{a}\right)\right]}^{1 / {m}_{n}}$

| **Function** | **Explanation** |
|---|---|
| ***ⁿ*(*CI*)*ₓ*** | The combination index for n drugs at x% inhibition |
| **(*Dₓ*)_{1-*n*}** | The dose of the sum of n drugs in combination that inhibit the cells by x% |
| ${\left\{\left[D\right]\right\}}_{j} / {\sum }_{1}^{n} \left.\left[D\right]\right\}$ | The proportionality of the dose of the n drugs that in combination inhibit the cells by x% |
| **(*Dₘ*)*ⱼ*{(*f_{aₓ}*)*ⱼ*/[1 - (*f_{aₓ}*)*ⱼ*]}^{1/*m^{j}*}** | The dose of individual drugs required to inhibit the cells by x% |
| **Dm** | The median-effect dose (antilog of the x-intercept of median-effect plot) |
| **fₓ** | The fractional inhibition at x% inhibition and m is the slope of the median-effect plot |
| **D** | The dose of the sum of n drugs in combination that inhibit the cells by x% |
| **D*x*** | The dose of individual drugs required to inhibit the cells by x% |

### EXPERIMENTAL RESULTS

### Individual cannabinoids

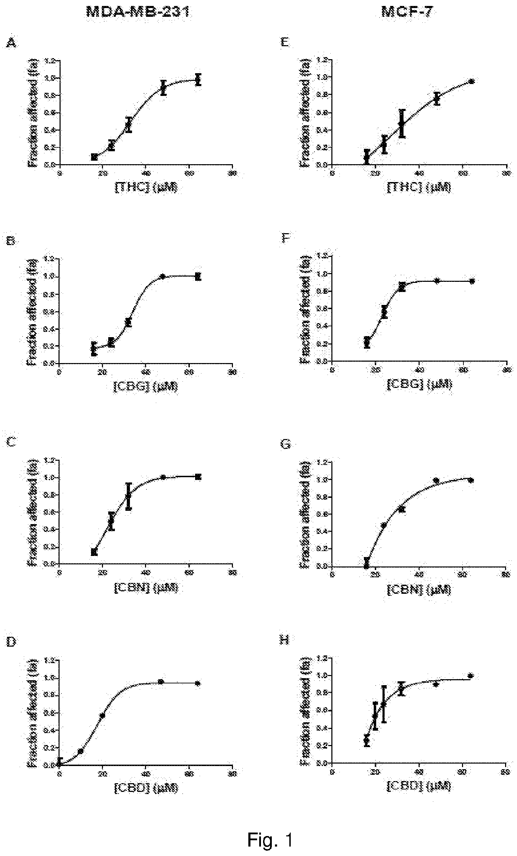

To evaluate whether the cannabinoid combinations were synergistic, the Chou and Talalay equation was used to determine the combination index (CI), where a CI value less than one is considered synergistic, CI equal to one is considered additive and a CI value of more than one represents an antagonistic interaction. To analyse various drug combinations, dose response curves can be constructed using two methods. The first method employs a constant ratio, whereby the drug combination is diluted serially to maintain the same ratio. The second method utilises a nonconstant ratio design, where one drug's concentration is kept constant, while the second drug's concentration decreases. The construction of dose response curves using a constant ratio design is recommended (Chou, 2006b). For the CI value to be determined, several parameters of the individual cannabinoids and combinations need to be known. These parameters are obtained from the linearisation of dose response curves to median-effect plots. Using the recommended constant ratio design method, dose response curves were constructed for individual cannabinoids in the MDA-MB-231 and MCF-7 breast cancer cell lines (Figure 1).

Referring to Figure 1, A - D represent dose response curves of THC, CBG, CBN and CBD respectively in MDA-MB-231 cells; E - H represent dose response curves of THC, CBG, CBN and CBD in MCF-7 cells. Cells were treated for 48 hours after which cell viability was measured using the MTT assay at 0.5 mg/ml. Fraction affected was calculated relative to DMSO vehicle control. Error bars represent SEM (n=3).

All cannabinoids were screened at the same concentration range (16 - 64 µM). Based on the dose response curves (Figure 1), it was evident that THC was the least cytotoxic cannabinoid since it had not reached the top plateau against both cell lines. It was therefore expected that the inhibitory concentrations (IC values) of THC would be the highest. To calculate the IC values, median effect plots had to be constructed. To obtain the median effect parameters, the dose response curves were linearised by plotting the logarithm of the drug concentration (log dose) on the x-axis and for the y-axis, the fraction affected (fa) was subtracted from one (1 - Fa = Fu) to obtain the fraction unaffected (fu). The Fa was then divided by the Fu, and the logarithm of the resulting fraction was calculated (log Fa/Fu).

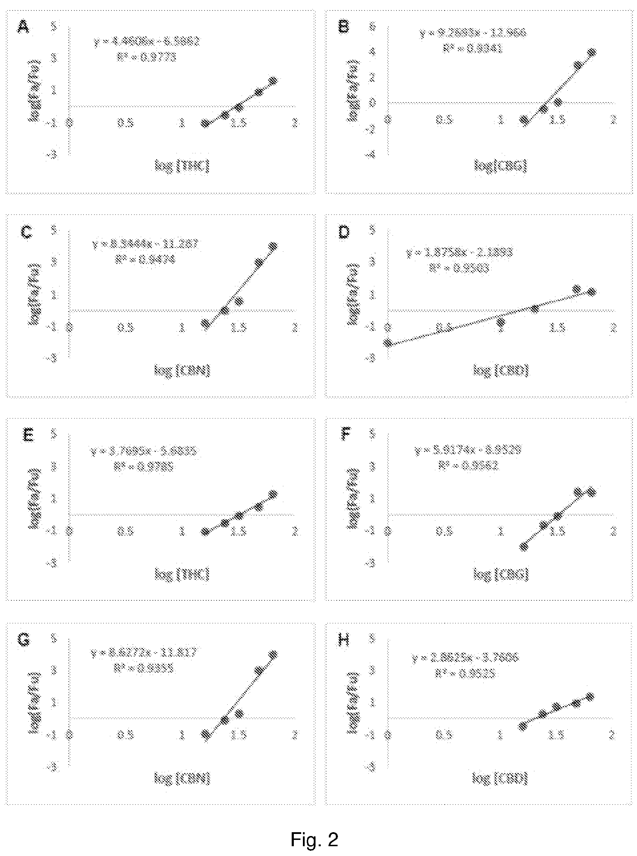

This conversion was used for each data point in the dose response curve. This generates a linear median-effect plot (Figure 2) fitted with the equation: y = mx + c, where y is the dependent variable, m is the slope and c is the y-intercept. In Figure 2, (A-D) is THC, CBG, CBN and CBD in the MDA-MB-231 cell line, and (E-H) is THC, CBG, CBN and CBD in MCF-7 cell line. Cells were treated for 48 hours after and cell viability was subsequently measured using the MTT assay at 0.5 mg/ml. Fraction affected was calculated relative to DMSO vehicle control (n=3).The degree of how well the regression prediction fits the experimental data, was measured using the statistical R² value. Using the linear equations and the R² value generated through the transformation of dose response curves to median-effect plots, information on the following median-effect parameters were obtained: the slope (m), potency (Dm) and conformity (r) value (Table 1). The r value corresponds to the R² value which ranges between 0 - 1. The closer the R² value is to 1, the better the data conforms to the regression model. The R² value was calculated using the RSQ function in Excel.

**Table 1: Median-effect parameters determined for individual cannabinoids in breast cancer cells MDA-MB-231 and MCF-7.**

| **Cell line** | **Cannabinoid** | ***Parameters^{#}*** | | | | |
|---|---|---|---|---|---|---|
| | | **m** | **r** | ***IC₅₀*** | **Dm (µM) *IC₇₅*** | ***IC₉₀*** |
| **MDA-MB-231** | *THC* | 4.46 | 0.99 | 29.96 | 38.33 | 49.03 |
| | *CBG* | 9.08 | 0.93 | 24.95 | 28.15 | 31.77 |
| | *CBN* | 8.34 | 0.94 | 22.52 | 25.69 | 29.31 |
| | *CBD* | 1.88 | 0.96 | 14.69 | 26.39 | 47.41 |
| **MCF-7** | *THC* | 3.77 | 0.98 | 32.21 | 43.11 | 57.70 |
| | *CBG* | 5.38 | 0.96 | 26.88 | 32.97 | 40.45 |
| | *CBN* | 8.63 | 0.95 | 23.43 | 26.61 | 30.22 |
| | *CBD* | 2.86 | 0.94 | 20.59 | 30.23 | 44.37 |

| | | | | | | |
|---|---|---|---|---|---|---|
| ^{#} Parameters consist of the slope (m), potency (Dm) and the linear correlation coefficient of the median effect plot (r). | | | | | | |

When considering the potency levels at IC₅₀, IC₇₅ and IC₉₀ levels for each of the four cannabinoids tested across the MDA-MB-231 and MCF-7 cell lines, it is noted that the MDA-MB-231 (triple-negative subtype) cell line predominantly had lower potency levels when compared to the MCF-7 cells, with the exception of CBD at IC₉₀. Despite the differences between the two cell lines, the order of potency (IC₅₀) of the cannabinoids remained the same, with a potency order starting from most potent: CBD>CBN>CBG>THC.

At IC₇₅ and IC₉₀, there was a shift in the cannabinoid potency order, with CBN becoming the most potent cannabinoid, across both cell lines. The shift in the potency of the cannabinoids at different inhibitory concentrations can be explained by the shape of the dose response curve. For example, an increase in the sigmoidal shape (m>1) of the curve would correlate to a lower concentration required to induce greater growth inhibition (Figure 1). The degree of sigmoidicity of the dose response curves are better represented by the slope (m) value of the median-effect plots for the MDA-MB-231 and MCF-7 (Figure 2) cell lines. An increase in sigmoidicity correlates to an increase in the slope of the median-effect plot (Table 1).

The MDA-MB-231 cell line is classified as a triple negative breast cancer (TNBC) cell line, meaning it doesn't express the oestrogen, progesterone or HER2 receptors. Consequently, the therapeutic options are limited in comparison to the hormone receptor positive breast cancers, represented by the MCF-7 cell line. TNBC cells are also characterised by high proliferation rates and metastasis. As a result, numerous attempts are made to identify molecular targets to treat TNBC cell. Therefore, an increased susceptibility to cannabinoid treatment is a promising result for the MDA-MB-231 cell line. Studies have reported that the MDA-MB-231 cells are more sensitive to the effects of THC, CBD and CBG; however, there are a limited amount of studies investigating the anti-proliferative effects of CBN in both the MDA-MB-231 and MCF-7 cell lines. Despite the increased sensitivity of the MDA-MB-231 cells to cannabinoid treatment, the triple-negative breast cancer cell lines have the worst prognosis of all breast cancer subtypes (Rodler *et al.,* 2010). Therefore, the lower inhibitory concentrations of the individual cannabinoids observed in the MDA-MB-231 cell line (Table 1) when compared to the MCF-7 cell line, may not be as promising.

The ICso values of individual cannabinoids were then used to combine the cannabinoids at double their respective IC₅₀:IC₅₀ concentrations, i.e. the two cannabinoid combination between THC and CBD in the MDA-MB-231 cell line was combined at IC₅₀:IC₅₀ (60 µM:30 µM). The parameters for THC, CBG, CBN and CBD were determined in the MDA-MB-231 and MCF-7 cell lines (Table 1).

### Two cannabinoid combinations with a constant ratio design

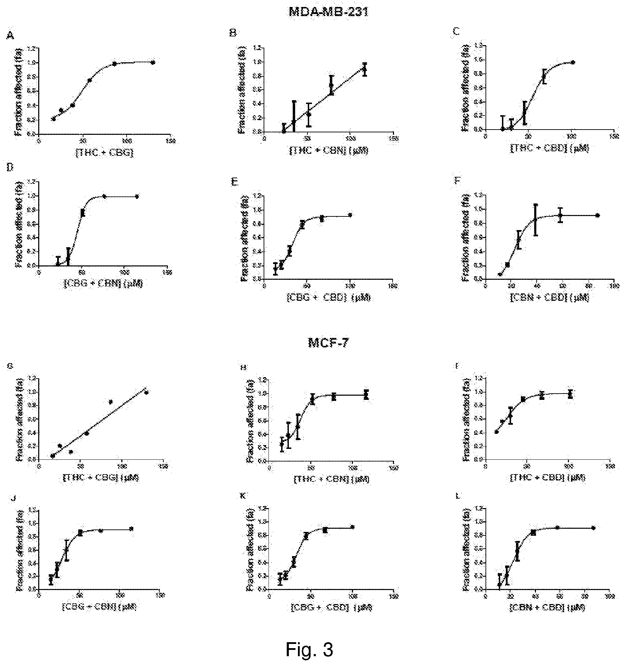

Once the parameters for the individual cannabinoids were established, the parameters for the cannabinoid combinations were determined using dose-response curves (Figure 3). Referring to Figure 3, cannabinoids were combined in all possible permutations for the two cannabinoids in the MDA-MB-231 cell line, A) THC and CBG, B) THC and CBN, C) THC and CBD, D) CBG and CBN, E) CBG and CBD and F) CBN and CBD; and the MCF-7 cell line G) THC and CBG, H) THC and CBN, I)THC and CBD, J) CBG and CBN, K) CBG and CBD and L) CBN and CBD. Cells were treated for 48 hours after which cell viability was measured using the MTT assay at 0.5 mg/ml. Fraction affected (fa) was calculated relative to DMSO vehicle control. Error bars represent SEM (n=3). There are two methods of evaluating drug-drug interactions. The first method quantitatively evaluates drug interactions using the Chou and Talalay combination theorem and describes the degree of the interaction, i.e. slight to very strong synergism (Chou, 2010). The second is the isobologram method, which qualitatively illustrates the interaction between drugs and only describes whether a drug interaction is synergistic, additive or antagonistic, but not the degree of the interaction. This study utilised the quantitative combination theorem, which provided more information regarding the degree of each antagonistic, additive or synergistic interaction. As recommended by Chou (2006), the cannabinoids were added in equipotent ratios at double the ICso concentration of each cannabinoid in the combination. These combinations were serially diluted to create dose-response curves in the MDA-MB-231 and MCF-7 cell lines (Figure 3).

All the cannabinoid combinations evaluated, displayed typical sigmoidal dose response curves, with the exception of the THC:CBG combination. This was unexpected since the THC:CBG combination consisted of the highest molar two-cannabinoid concentration. This suggested that THC and CBG induced their anti-proliferative effects via mechanisms that are diminished when they are in combination. A possible explanation could be via competition for the same binding site on the cannabinoid receptors, through which they exert their anti-proliferative effects. Studies revealed that CBG competed for binding of the synthetic cannabinoid WIN-55, 212-3 (structurally similar to THC) to the CB₂ receptor, but not the CB₁ receptor (Navarro *et al.,* 2018). In the presence of both receptors, CBG has a higher affinity for the CB₂ receptor. Therefore, it could be possible that CBG is competing with THC for binding to the CB₂ receptor, reducing the anti-proliferative effects exerted by the THC:CBG combination.

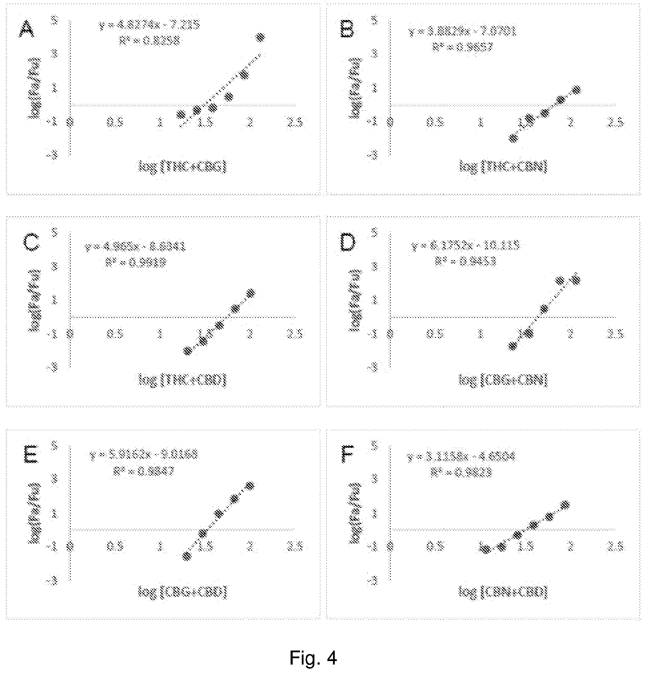

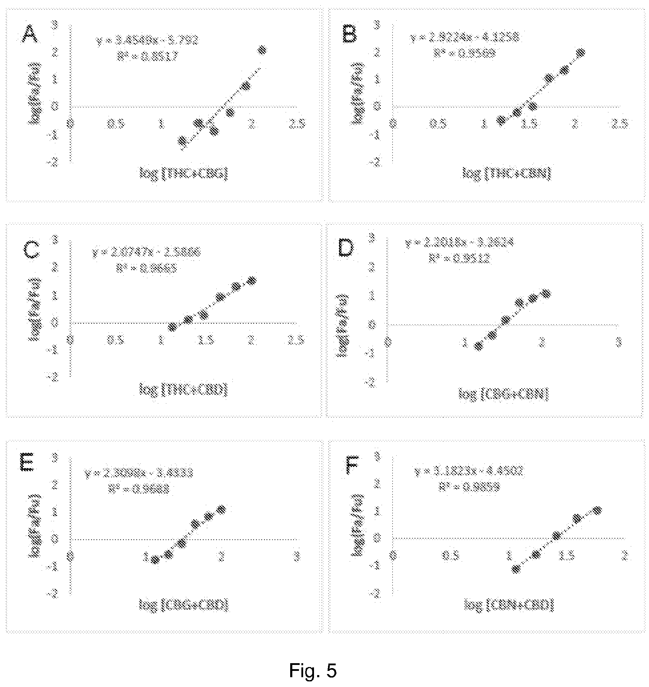

Median-effect plots were constructed for the MDA-MB-231 (Figure 4) and MCF-7 (Figure 5) cell lines, respectively, based on the respective dose response curve of two cannabinoid combinations. The linear equations were subsequently used to determine the median-effect parameters required to calculate the combination indices (CI) for the two cannabinoid combinations. Referring to Figure 4, cannabinoids were combined in all possible permutations of two cannabinoids - A) THC and CBG, B) THC and CBN, C) THC and CBD, D) CBG and CBN, E) CBG and CBD and F) CBN and CBD. Cells were treated for 48 hours after which cell viability was subsequently measured using the MTT assay at 0.5 mg/ml. Fraction affected was calculated relative to DMSO vehicle control (n=3). Fa - Fraction affected; Fu - Fraction unaffected.

The linearization of dose response curves to median-effect plots were also completed in the MCF-7 cell line (Figure 5). Referring to Figure 5, cannabinoids were combined in all possible permutations of two cannabinoids - A) THC and CBG, B) THC and CBN, C) THC and CBD, D) CBG and CBN, E) CBG and CBD and F) CBN and CBD. Cells were treated for 48 hours; cell viability was determined using the MTT assay at 0.5 mg/ml. Fraction affected (fa) was calculated relative to DMSO vehicle control (n=3). Fa - Fraction affected; Fu - Fraction unaffected.

Prior to quantifying the interaction between the two cannabinoid combinations using the acquired parameters, it is important to first evaluate how well the regression model fits the data, to accurately conclude on the trends observed. According to Chou (2006), an r value of 0.95 - 1 is required for in vitro studies. The majority of the two-cannabinoid combinations complied with this prerequisite (Figure 4 and Figure 5). Exceptions were observed for the combination of THC with CBG in both breast cancer cell lines, which had r values of 0.826 and 0.852 in MDA-MB-231 (Figure 4) and MCF-7 cells (Figure 5), respectively. This highlighted one of the limitations of using the Chou and Talalay method for analysing drug interactions. The use of log-linearisation may lead to poor model fits (as observed for the THC:CBG combination), which could be addressed through the use of non-linear regression models. This could allow for greater flexibility, when fitting appropriate curves to the data, ultimately improving the validity of the conclusions reached (synergism, additivity or antagonism) (Ashton, 2015). However, the combination index theorem only relies on parameters obtained through log-linearisation of the experimental data points. Therefore, the Dm values for the THC:CBG combination were not determined, as the linear model does not accurately fit the data, which would lead to the inaccurate determination of the Dm values.

An example of how the median-effect parameters were determined from the median-effect plots, using the cannabinoid combination CBG:CBN at IC₉₀ in the MCF-7 cell line is illustrated below:
*Slope of the median-effect plot:* $m = \frac{{y}_{2} - {y}_{1}}{{x}_{2} - {x}_{1}} = \frac{1 - 0.5}{1.936 - 1.708} = \frac{0.5}{0.228} = 2.2$
*Potency (Dm) value:*
   Dm = x-intercept of the linear median-effect plot
   Therefore, following the linear equation: y = mx + c
   Where, $y = log \left(\frac{0.9}{1 - 0.9}\right)$; m = 2.2018; c = - 3.2624; x = log[CBG+CBN] $x = \left(\frac{y + c}{m}\right)$ $x = \left(\frac{0.954 + 3.2624}{2.2018}\right) = 1.915$ $∴ {IC}_{90 \left(CBG+CBN\right)} = {10}^{x} = {10}^{1.915} = 82.2 μM$

The median-effect parameters were calculated for the two cannabinoid combinations (Table 2), following the same calculations illustrated in the example. When evaluating the potency (Dm) values of the various two cannabinoid combinations (Table 2), it was expected that the potency (IC values) of the cannabinoid combinations would be lower in the MDA-MB-231 cell line, when compared to the MCF-7 cell line, resembling the trend observed for the Dm values obtained for the individual cannabinoids (Table 1). This was not observed when looking at the various IC values (IC₅₀, IC₇₅ and IC₉₀) of the two-cannabinoid combinations. The MCF-7 cells displayed lower IC values for all IC values when compared to the MDA-MB-231 cells, with the exception of the CBG:CBD combination (Table 6).

**Table 2: Median-effect parameters for cannabinoid combinations consisting of THC, CBG, CBN, and CBD in breast cancer cells.**

| **Cell line** | **Combination** | ***Parameters^{#}*** | | | | |
|---|---|---|---|---|---|---|
| | | **m** | **r** | ***IC₅₀*** | **Dm (µM) *IC₇₅*** | ***IC₉₀*** |
| **MDA-MB-231** | *THC and CBG* | 4.827 | 0.826 | - | - | - |
| | *THC and CBN* | 3.883 | 0.966 | 66.20 | 87.85 | 116.58 |
| | *THC and CBD* | 4.965 | 0.992 | 54.83 | 68.41 | 85.35 |
| | *CBG and CBN* | 6.175 | 0.945 | 43.46 | 51.92 | 62.03 |
| | *CBG and CBD* | 5.916 | 0.985 | 33.43 | 40.25 | 48.46 |
| | *CBN and CBD* | 3.116 | 0.982 | 31.08 | 44.21 | 62.90 |
| **MCF-7** | *THC and CBG* | 3.455 | 0.852 | - | - | - |
| | *THC and CBN* | 2.922 | 0.957 | 25.81 | 37.59 | 54.74 |
| | *THC and CBD* | 2.178 | 0.972 | 17.65 | 29.97 | 50.90 |
| | *CBG and CBN* | 2.202 | 0.951 | 30.32 | 49.94 | 82.24 |
| | *CBG and CBD* | 2.310 | 0.968 | 36.24 | 59.68 | 98.29 |
| | *CBN and CBD* | 3.182 | 0.986 | 25.04 | 35.36 | 49.94 |

| | | | | | | |
|---|---|---|---|---|---|---|
| ^{#}Parameters consist of slope (m), potency (Dm) and the linear correlation coefficient of the median effect plot (r). | | | | | | |

A possible explanation for the enhanced anti-proliferative effects in the MCF-7 cell line could be the expression of the HER2 receptor, which is absent in the MDA-MB-231 cell line. Studies have shown that the HER2 receptor forms heterodimers with CB₂, facilitating the proliferative effects mediated by HER2, via pathways including the phosphatidylinositol 3-kinase/protein kinase B/mammalian target of rapamycin (PI3K/Akt/mTOR) pathway. The binding of cannabinoids to CB₂, disrupts the HER2/CB₂ heterodimer formation, inhibiting the PI3K/Akt pathway (Blasco-Benito *et al.,* 2019, Caffarel *et al.,* 2010). Since all four cannabinoids have the ability to bind to the CB₂ receptor (Pertwee *et al.,* 2008), their anti-proliferative effects in the MCF-7 cells would be amplified when cannabinoids are combined.

The three combinations with the highest cytotoxicity in the MDA-MB-231 cells (based on the IC₅₀, IC₇₅ and IC₉₀), ranging from the most cytotoxic to the least were: CBN:CBD, CBG:CBD and CBG:CBN (Table 2). These combinations excluded the cannabinoid, THC. This suggested that the presence of THC in the combination decreased the potency of the cannabinoid combinations in the MDA-MB-231 cell line. The three combinations with the lowest IC values in the MCF-7 cell line, ranging from the lowest to the highest were: THC:CBD, CBN:CBD, and THC:CBN (Table 2). Unlike in the MDA-MB-231 cell line, two of the three most potent two cannabinoid combinations in the MCF-7 cell line included THC. It couldn't be established whether the three most potent two cannabinoid combinations in the MCF-7 cell line included THC, since the regression model for the THC:CBG combination did not adhere to the prerequisite of an r value of at least 0.95.

The difference in endocrine sensitivity of the two cell lines could possibly explain why cannabinoid combinations containing THC were the most cytotoxic in the MCF-7 cells, while THC-containing combinations were the least cytotoxic in the MDA-MB-231 cells. Oestrogen receptor positive breast cancers often depend on oestrogenic activity for their enhanced proliferation rate. The oestrogenic effects are mediated via two nuclear receptors, ERα and ERβ. Upon activation of the ERα by 17β oestradiol (E2), cyclin D1 and cyclin-dependent kinases (cdks) are upregulated, which facilitates cell cycle progression from the G1 to the S phase through hyperphosphorylation of the retinoblastoma protein (Rb). The ERβ is a repressor of the ERα-mediated proliferation, through the formation of heterodimers with the ERα and homodimers of the ERβ. THC (1, 2.5, 5, 10, 25 and 50 µM) has been reported to suppress the ERα-mediated proliferation of MCF-7 cells through the upregulation of the ERβ repressor, in the absence of direct interaction of THC with the ERβ ligand binding site (Takeda *et al.,* 2013). As such, the presence of THC in the combination of two cannabinoids possibly enhanced the anti-proliferative effect via the disruption of the ERα-mediated proliferation. This could explain why the presence of THC enhanced the anti-proliferative activity of the cannabinoid combinations in the MCF-7 cell line but was less effective in the ER-negative MDA-MB-231 breast cancer cell line (Table 2).

After the median-effect parameters of two cannabinoid combination were determined, the combination indices were evaluated of all combinations that adhered to the prerequisites (Table 3). Since the CI value is dependent on the inhibitory concentrations of the individual cannabinoids and cannabinoid combinations, at a given percentage inhibition, the CI value could vary depending on the inhibitory concentration at which it is calculated. Therefore, the results indicated the CI values at IC₅₀, IC₇₅ and IC₉₀ (Table 3). The higher potency values, i.e. 90% have a greater therapeutic relevance in the treatment of cancer since the majority of the cancer cell population would be affected by the treatment; therefore, only the CI at 90% was described (Table 3).

**Table 3: Combination index values determined for two cannabinoid combinations in the breast cancer cell lines using the median-effect plot parameters.**

| **Cell line** | ***Combination*** | **Combination Index (CI*)** | | | **Description of CI at 90%** |
|---|---|---|---|---|---|
| | | **50%** | **75%** | **90%** | |
| **MDA-MB-231** | *THC and CBN* | 2.52 | 2.78 | 3.07 | Antagonism |
| | *THC and CBD* | 2.50 | 2.07 | 1.76 | Antagonism |
| | *CBG and CBN* | 1.86 | 1.95 | 2.05 | Antagonism |
| | *CBG and CBD* | 1.71 | 1.49 | 1.36 | Moderate antagonism |
| | *CBN and CBD* | 1.68 | 1.70 | 1.80 | Antagonism |
| **MCF-7** | *THC and CBN* | 0.93 | 1.10 | 1.32 | Moderate antagonism |
| | *THC and CBD* | 0.75 | 0.85 | 0.98 | Nearly additive |
| | *CBG and CBN* | 1.20 | 1.67 | 2.34 | Antagonism |
| | *CBG and CBD* | 1.43 | 1.63 | 1.90 | Antagonism |
| | *CBN and CBD* | 1.28 | 1.34 | 1.44 | Moderate antagonism |

| | | | | | |
|---|---|---|---|---|---|
| CI signifies the combination index at 50%, 75% and 90% inhibition of the cell population where CI = [(D)1 / Dx)1] + [(D)2/ Dx)2], where Dx = Dm[fa/(1-fa)]1/m. A CI < 1 indicates synergism, CI = 1 indicates an additive effect and CI > 1 antagonism. | | | | | |

At 90%, the majority of the combinations were antagonistic (CI > 1), ranging from moderate to complete antagonism. THC and CBD were the only combination that displayed promise in obtaining a synergistic interaction against the MCF-7 cell lines. THC and CBD displayed an additive interaction at an inhibitory concentration of 90%; however, at 50% and 75% growth inhibition, the combination was moderately synergistic with respective CI values of 0.75 and 0.85.

Since THC had the highest ICso of all the cannabinoids (Table 1), and a low ICso value in combination, the calculated CI value was low. For the other cannabinoids, the ICso of the individual cannabinoids were low, therefore the CI value increased, even if the ICso value of the combination remained low. Therefore, to obtain synergistic combinations (CI<1) at the higher effect levels, *e.g.* 90%, the denominator (individual cannabinoid concentration) has to increase, or the numerator (combined cannabinoid concentration) has to decrease. Since the inhibitory concentration of individual cannabinoids could not change, the only alternative to obtain low CI values was to attain cannabinoid combination(s) with low inhibitory concentrations, while retaining its efficacy, which is the ideal scenario when considering the use of combination therapy.

With regards to the two cannabinoid combinations tested (Table 3), THC and CBD was the only synergistic combination at 50% and 75% growth inhibition, while no synergistic interactions were observed at 90% growth inhibition (Table 3). The combination between THC and CBD was also tested at different ratios of THC:CBD at 9:1 and 1:3. The THC:CBD ratio of 9:1 yielded synergism at all three potency levels (50%, 75% and 90%), while the ratio of 1:3 yielded no synergism. Although the combination of cannabinoids remained the same (THC and CBD), some outcomes were synergistic, while others were antagonistic. This illustrated how the CI value changed depending on the ratio at which the cannabinoids were combined. This data therefore suggested that cannabinoids should be combined at different ratios, to obtain potential synergistic interactions in both MDA-MB-231 and MCF-7 cell lines at 90% growth inhibition. To systematically test a wide range of cannabinoid ratios, a checkerboard screening method was designed, permitting the combination of four cannabinoids (THC, CBG, CBN and CBD), at different ratios.

### Four cannabinoid combinations: non-constant ratio design

The checkerboard assay was specifically designed to contain high THC concentrations, which showed promise in the MCF-7 cells, as well as low THC concentrations, since THC decreased the potency of the combinations in the MDA-MB-231 cells (Table 2). Studies have reported the protective role of CBD against the psychotic activity of THC; however, the minimum concentration required for CBD to exert its protective effects were not specified (Niesink and van Laar, 2013). Therefore, the checkerboard assay was designed to contain varying ratios of a high THC to CBD and a high CBD to THC ratio (Figure 6).

THC is susceptible to heat and oxidation, which converts THC to its primary degradation product, CBN. Degradation has been reported to occur even at low temperatures ranging between 4 - 22 °C. As such, the higher the THC content in a recreational cannabis strain, the higher the CBN content would be when the recreational strain degrades. Therefore, the layout of the checkerboard assay was selected to include cannabinoid ratios with high CBN concentrations to evaluate the potential for degraded cannabis extracts. Finally, the limited studies evaluating the anti-proliferative effects of CBG also prompted the checkerboard assay to include high CBG ratios to evaluate the potential for high CBG containing cannabinoid therapies in breast cancer (Figure 6). After mixing four cannabinoids at various concentrations in each respective well, several four cannabinoid ratios were generated (Figure 7).

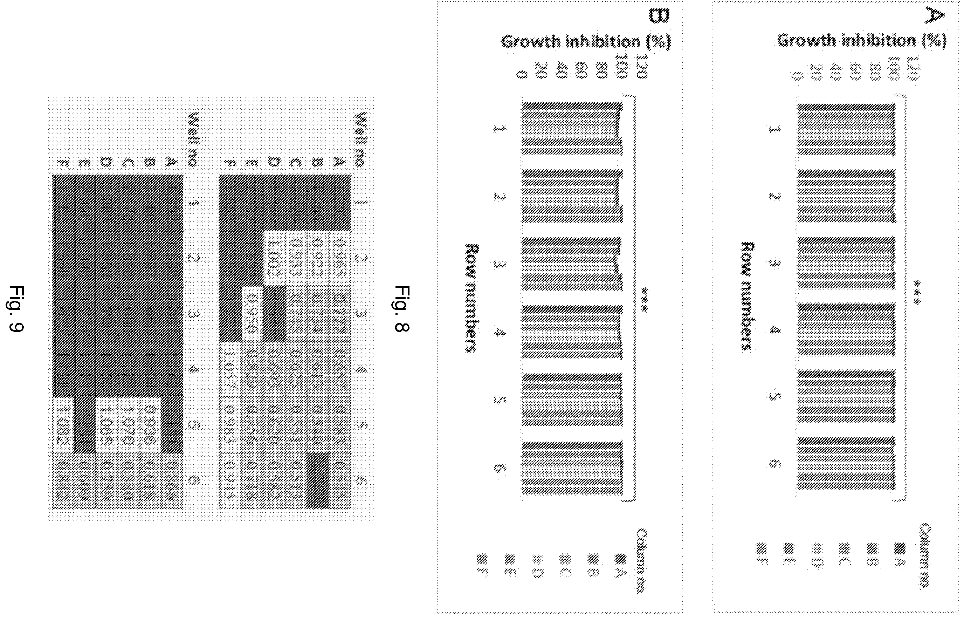

These 48 four-cannabinoid ratios were screened against the MDA-MB-231 and MCF-7 cell lines (Figure 8). The checkerboard assay yielded potent cannabinoid combinations, with highly significant growth inhibition percentages ranging between 99 - 100% in MDA-MB-231 cells, and 94 - 100% in the MCF-7 cells (Figure 8), under the conditions tested. In Figure 8, A) represents the MDA-MB-231 and B) represents the MCF-7 cell line, cells were exposed to combinations of the four cannabinoids at different ratios for 48 hours, after which, the MTT assay (0.5mg/ml) was performed to determine the growth inhibition induced relative to the DMSO vehicle control. Error bars represent SEM (n=3). **p<0.0001 relative to DMSO vehicle control. A - F represent column numbers and 1 - 6 represent row numbers corresponding to the cannabinoid ratios described in Figure 7. The next step was to determine the combination indices for these combinations. This requires that the median-effect parameters are known. Since each ratio was represented by a single data point and not a dose response curve, the median-effect parameters and subsequently the CI values could not be determined at a given effect level (50%, 75% or 90%). Nonetheless, the CI value could be determined at the experimental effect level induced by the cannabinoid combination, provided that the median-effect parameters for the individual cannabinoids are known (Chou, 2006a).

Using the regression models of the individual cannabinoids, the concentration required for the individual cannabinoids to induce the same percentage growth inhibition as the combination can easily be determined, provided the percentage growth inhibition induced by the cannabinoid combination, falls within the range covered by the regression models of the individual cannabinoids. It was important that the individual cannabinoids induced the same level of growth inhibition as the cannabinoid combinations tested, since any predictions made beyond this range, would yield inaccurate results, especially since cannabinoids are known to display biphasic effects.

The CI values were obtained for the four-cannabinoid combinations screened against the MDA-MB-231 and the MCF-7 cell lines to provide an indication of which combinations to construct dose response curves for, as a means of obtaining accurate CI values, that are obtained from values that fall within the regression models of the individual cannabinoids (Figure 9). In Figure 9, (Top) represents MDA-MB-231 and (Bottom) represents MCF-7 cell lines. CI signifies the combination index at 50%, 75% and 90% inhibition of the cell population where CI = [(D)1 / Dx)1] + [(D)2/ Dx)2], where Dx = Dm[fa/(1-fa)]1/m. A CI < 1 indicates synergism, CI = 1 indicates an additive effect and CI > 1 antagonism.

Surprisingly, the checkerboard assay yielded 17 potentially synergistic four-cannabinoid ratios against the MDA-MB-231 cell line (Figure 9), which previously displayed no synergistic interactions when two cannabinoid combinations were tested (Table 3).

To reduce the costs of the study, only half of the synergistic combinations were selected for further evaluation, which included the 8 potentially synergistic four-cannabinoid combinations with the lowest CI values. The most promising synergistic interactions (CI values ranged from 0.513 - 0.657) obtained against the MDA-MB-231 cells, were the ratios associated with wells **A4** - **A6, B4** - **B5** and **C4** - **C6** in the checkerboard assay (Figure 9). In the MCF-7 cell line, the checkerboard assay yielded 6 potentially synergistic ratios (present in column 6, rows A to F), with CI values ranging from 0.38 - 0.88. Based on the checkerboard assay design (Figure 6), it was evident that no synergistic interactions were obtained when the majority of the cannabinoids in the combination consisted of a high THC, CBG or CBN content. Several synergistic interactions were obtained for ratios consisting of a high CBD content (Figure 6; Figure 9).

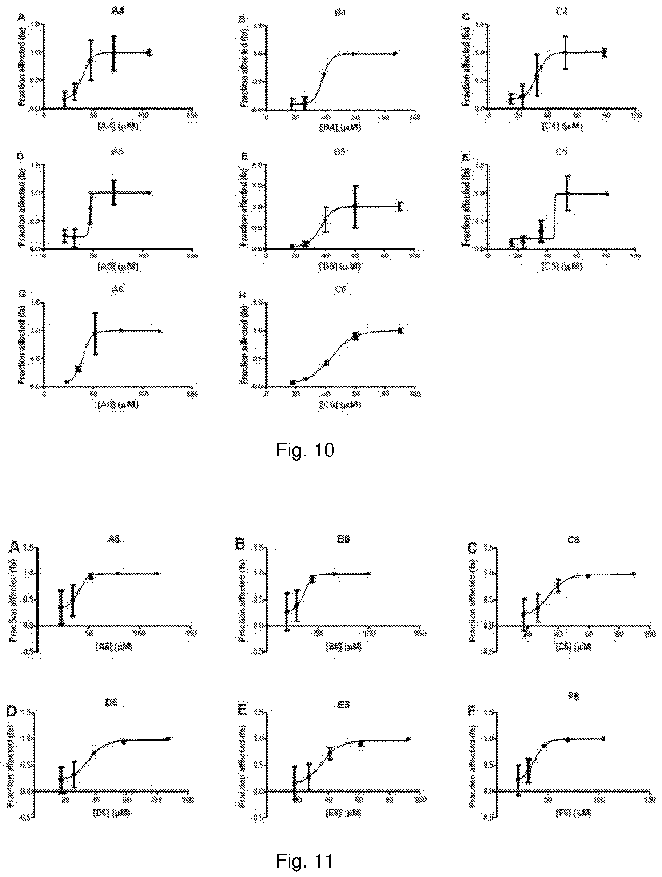

One of the benefits of CBD being the most cytotoxic cannabinoid in the various combinations (Table 1, Figure 9), is its non-psychotropic nature. This property increases the therapeutic relevance of CBD in alternative cancer treatments. Although CBD displayed potent anti-proliferative activity in the checkerboard assay, the next stage of the study was to determine if the combinations were synergistic when the CI values were determined within the regression models of the individual cannabinoids. In the example described previously where the CI value for **C5** was determined at 97,8% growth inhibition, three of the four cannabinoid concentrations calculated, fell outside the range covered by the regression models of the individual cannabinoids, leading to the calculation of inaccurate CI values. To solve this problem, dose response curves were prepared for the preliminary synergistic cannabinoid combinations in the MDA-MB-231 cell line (Figure 10) (**A4**, **A5, A6, B4, B5, C4, C5** and **C6**; corresponding to the well numbers in Figure 9a) and MCF-7 cell line (Figure 11) (**B6, C6, D6** and **E6,** corresponding to well numbers in Figure 9b).

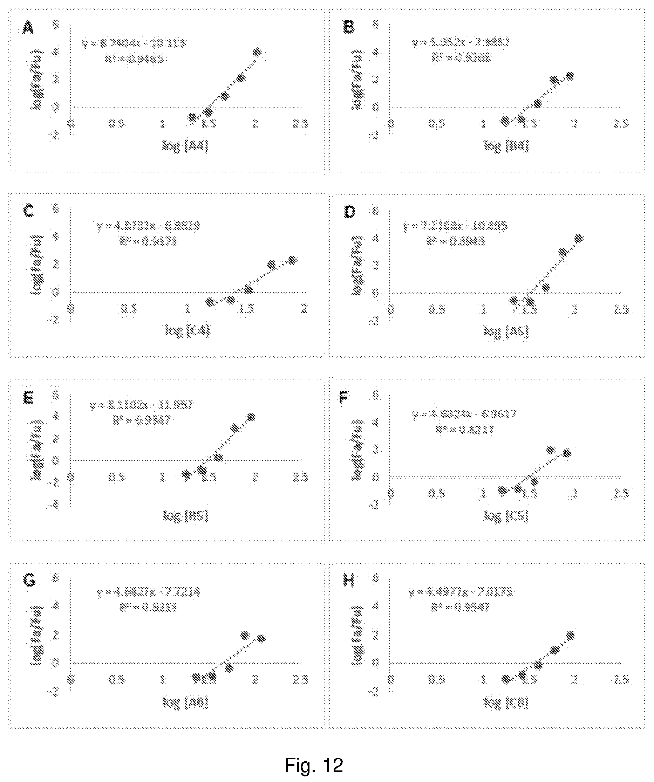

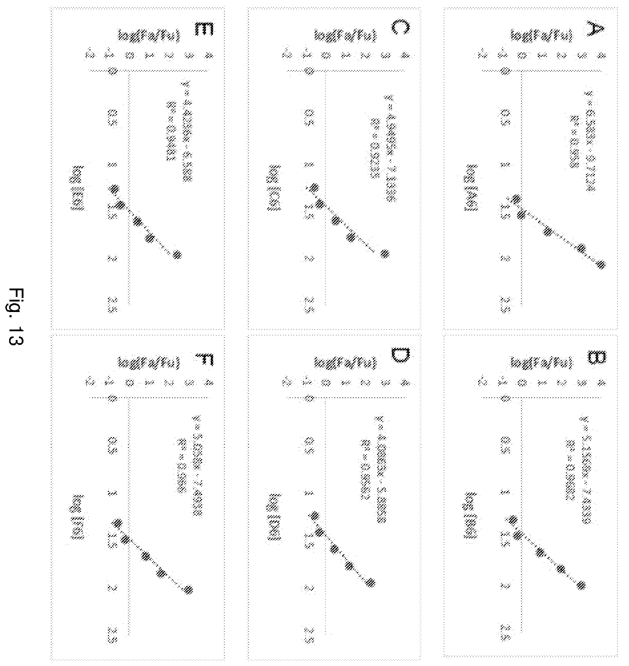

Dose response curves were constructed for the preliminary synergistic combinations. The maximum concentration of each combinations was obtained using the concentrations indicated in Figure 6, which was subsequently diluted 1.5 fold to create six-point dose response curves. All the dose response curves displayed typical sigmoidal shapes (Figure 10 and Figure 11). The dose response curves were linearised to construct median-effect plots for MDA-MB- 231 (Figure 12) and MCF-7 cells (Figure 13), respectively. Referring to Figure 12, A) **A4,** B) **B4**, C) **C4,** D) **A5,** E) **B5,** F) **C5,** G) **A6** and H) **C6**. Each code corresponded to a specific well in the checkerboard layout with specific ratios comprising of the four cannabinoids. Cells were treated for 48 hours and cell growth inhibition was measured using the MTT assay at 0.5 mg/ml. Fraction affected (fa) was calculated relative to DMSO vehicle control SEM (n=2). Fa - Fraction affected; Fu - Fraction unaffected. Referring to Figure 13, A) **A4,** B) **B6**, C) **C6,** D) **D6,** E) **E6** and F) **F6**. Each code corresponded to a specific well in the checkerboard layout. Cells were treated for 48 hours, after which cell growth inhibition was determined using the MTT assay at 0.5 mg/ml. Fraction affected (fa) was calculated relative to DMSO vehicle control (n=3).

The majority of the parameters obtained for the four-cannabinoid combinations complied to the prerequisite of an r value of more than 0.95. The combinations that did not comply, included **A5** and **C5** in the MDA-MB-231 cell line. The other combinations, **B4, C4** and **B5** in the MDA-MB-231 cell line and **C6** in the MCF-7 cell line were considered for further analysis, since the r values obtained were approaching the required value of 0.95. The linear equations from the median-effect plots of MDA-MB-231 (Figure 12) and MCF-7 (Figure 13) were used to determine the median-effect parameters (Table 4). The CI values were then determined at 90% growth inhibition (Table 4), which fell within the regression model covered by the individual cannabinoids, increasing the reliability and accuracy of the results.

**Table 4: Median-effect parameters and combination index values determined for four cannabinoid combinations in breast cancer cells.**

| **Cell line** | ***Combination*** | **Parameters** | | | | | **CI at 90%** | **Description of CI* at 90%** |
|---|---|---|---|---|---|---|---|---|
| | | **m** | **r** | **Dm (µM)** | | | | |
| | | | | **IC₅₀** | **IC₇₅** | **IC₉₀** | | |
| **MDA-MB-231** | *A4* | 6.740 | 0.946 | 31.66 | 37.26 | 43.86 | 1.023 | Nearly additive |
| | *B4* | 5.384 | 0.921 | 31.00 | 38.02 | 46.62 | 1.143 | Slight antagonism |
| | *C4* | 4.873 | 0.918 | 25.49 | 31.93 | 40.01 | 1.032 | Nearly additive |
| | *A5* | 7.211 | 0.894 | - | - | - | - | - |
| | *B5* | 8.110 | 0.935 | 29.81 | 34.13 | 39.09 | 0.924 | Nearly additive |
| | *C5* | 4.682 | 0.911 | - | - | - | - | - |
| | *A6* | 4.752 | 0.978 | 42.16 | 53.12 | 66.94 | 1.147 | Slight antagonism |
| | *C6* | 4.498 | 0.955 | 36.32 | 46.37 | 59.20 | 1.477 | Antagonism |
| **MCF-7** | *A6* | 6.583 | 0.958 | 29.88 | 35.31 | 41.72 | 0.853 | Slight synergism |
| | *B6* | 5.157 | 0.968 | 27.64 | 34.20 | 42.32 | 0.854 | Slight synergism |
| | *C6* | 4.949 | 0.924 | 27.62 | 34.49 | 43.06 | 0.901 | Nearly additive |
| | *D6* | 4.086 | 0.948 | 27.57 | 36.07 | 47.20 | 1.150 | Slight antagonism |
| | *E6* | 4.424 | 0.966 | 30.85 | 39.55 | 50.70 | 1.313 | Moderate antagonism |
| | *F6* | 5.058 | 0.968 | 30.31 | 37.66 | 46.80 | 1.283 | |

| | | | | | | | | |
|---|---|---|---|---|---|---|---|---|
| CI signifies the combination index at 50, 75 and 90% inhibition of the cell population where CI = [(D)1 / Dx)1] + [(D)2 / Dx)2], where Dx = Dm[fa/(1-fa)]1/m. CI < 1, CI = 1 and CI > 1 indicates synergism, an additive effect or antagonism, respectively. | | | | | | | | |

In the MDA-MB-231 cells, all the cannabinoid combinations displayed either additive or antagonistic interactions at 90% growth inhibition (Table 4), despite the previously low combination indices identified (Figure 9). In the MCF-7 cells, **A6** and **B6** were the only combinations that displayed synergism at 90% growth inhibition with the same CI value of 0.85. The combinations selected for further studies were not only based on their synergistic interactions, but on their THC content. Studies have reported that THC is psychoactive (Koguel *et al.,* 2018, Casajuana Koguel *et al.,* 2018, Niesink and van Laar, 2013) and induces DNA damage in neuronal cells. Therefore, the potential use of cannabinoids as an alternative cancer therapy should limit the THC content, to reduce the chances of neuronal cell death, which may lead to the onset of neurodegenerative diseases. Based on the synergistic data obtained for the four cannabinoid combinations, only **B6** was selected for further studies, since it contained a lower THC content than **A6** (Figure 6). The remaining four combinations displayed antagonistic interactions, with the exception of **C6** in the MCF-7 cell line (Table 4), which displayed a nearly additive interaction (CI value of 0.901). The CI value for **C6** at 90% growth inhibition was additive, while the CI value at 91% growth inhibition was 0.886 (moderate synergism). Therefore, **C6** was also selected for further studies.

As previously mentioned, synergistic interactions reduce the required dose, minimises the potential risk of developing drug resistance, decrease the cost associated with therapy and minimises the potential toxicity to the host. These benefits were evaluated by calculating the dose reduction index and screening for toxicity in non-cancerous cells.

### Dose reduction index (DRI)

Dose reduction can be measured using the dose reduction index (Caba *et al.,* 2011, Chou, 2006a). The DRI represents the fold change, by which the concentration for each drug in combination can be reduced, in comparison to the concentration required by the individual drug, to induce the same growth inhibition. The DRI equation is the inverse relationship of the combination index equation; therefore, for the combination of two drugs, the DRI can be calculated as follows: $DRI = \frac{{\left({D}_{x}\right)}_{1}}{{\left(D\right)}_{1}} + \frac{{\left({D}_{x}\right)}_{2}}{{\left(D\right)}_{2}}$

A DRI greater than one is considered favourable. The DRI values obtained for **B6** and **C6** in the MCF-7 cell line were calculated at 50%, 75% and 90% growth inhibition (Table 5).

**Table 5: Concentrations of THC, CBG, CBN and CBD required individually and in combination to induce 50%, 75% and 90%. Dose reduction index values for synergistic four-cannabinoid combinations at various effect levels in MCF-7 cells.**

| **Growth inhibition** | **Required concentration of cannabinoids:** | **B6** | | | | **C6** | | | |
|---|---|---|---|---|---|---|---|---|---|
| | | THC | CBG | CBN | CBD | THC | CBG | CBN | CBD |
| 50% | Individual Combination DRI | 32.58 | 26.88 | 23.43 | 20.59 | 32.58 | 26.88 | 23.43 | 20.59 |
| | | 12.22 | 2.22 | 2.78 | 10.71 | 8.84 | 2.42 | 4.53 | 11.62 |
| | | 2.64 | 12.06 | 8.41 | 1.46 | 3.72 | 11.12 | 5.16 | 1.43 |
| 75% | Individual Combination DRI | 39.23 | 32.97 | 26.61 | 30.23 | 39.23 | 32.97 | 26.61 | 30.23 |
| | | 14.54 | 2.65 | 3.32 | 12.76 | 10.58 | 2.89 | 5.43 | 13.93 |
| | | 2.96 | 12.42 | 8.03 | 2.06 | 4.08 | 11.38 | 4.90 | 1.88 |
| 90% | Individual Combination DRI | 47.24 | 40.45 | 30.22 | 44.37 | 47.24 | 40.45 | 30.22 | 44.37 |
| | | 17.32 | 3.16 | 3.94 | 15.19 | 12.68 | 3.46 | 6.50 | 16.68 |
| | | 3.34 | 12.80 | 7.65 | 2.89 | 4.56 | 11.66 | 4.64 | 2.62 |

The inhibitory concentrations of **B6** and **C6** in the MCF-7 cell line (Table 4) were higher than the inhibitory concentrations of the individual cannabinoids with the exception of THC (Table 1). It should be noted that an ideal synergistic combination does not reduce the total dose of the combination (i.e. sum of the concentrations of THC, CBG, CBN and CBD comprising the combination) to less than the inhibitory concentration of the individual cannabinoids; however, the required dose of each cannabinoid in the combination, relative to the dose required for the individual cannabinoids, is reduced (Table 5). Each of the four cannabinoids comprising the combinations, **B6** and **C6** displayed favourable dose reduction indices (DRI>1) ranging between 1.46 - 12.80 and 1.43 - 11.66, respectively (Table 5). Based on these favourable DRI values, **B6** and **C6** may display minimised toxicity to the host.

### Toxicity in non-cancerous cells and white blood cells

The potential toxicity induced by the selected treatments (**B6** and **C6**) were evaluated in two ways: 1) screening in the normal breast cells, MCF-10A and 2) determining the cytotoxicity to white blood cells isolated from human volunteers (Figure 14). Referring to Figure 14, A) represents MCF-10A non-cancerous breast cells which were treated with the IC₉₀ of synergistic combinations which included **B6** (42 µM) and **C6** (43 µM) for 48 hours with camptothecin (5.7 µM) as the positive control. B) represents white blood cells which were treated with the IC₉₀ of synergistic combinations which included **B6** (42 µM) and **C6** (43 µM) for 24 hours with curcumin (90 µM) as the positive control. The percentage cell survival was determined relative to the DMSO vehicle control. Error bars represent SEM (n=3). *p<0.05, ***p<0.001 relative to DMSO vehicle control using one way ANOVA with post hoc Tukey test.

When comparing the effects of the synergistic combinations in the MCF-10A cells, it was observed that **B6** induced 55% inhibition, while **C6** only induced 4% inhibition. CBD had the highest concentration (17 µM) amongst the four cannabinoids used in the ratios **B6** and **C6.** CBD was previously shown to be the most potent cannabinoid of the four individual cannabinoids tested (Table 1) and the key cannabinoid present in the four cannabinoid combinations (Figure 9). Due to the high CBD concentration, it was expected that **B6** and **C6** induced growth inhibition in the MCF-10A cells since they contained the same CBD concentration; however a difference in the cytotoxicity induced by **B6** and **C6** against the MCF-10A cell line, was observed (Figure 14). The only difference between **B6** and **C6** was the THC (17 µM and 13 µM, respectively) and CBN content (4 µM and 6 µM, respectively) while the CBD and CBG concentrations were the same (Table 5). The difference in cytotoxicity induced by **B6** and **C6,** could be explained by the difference in their cannabinoid concentrations, with effects mediated via nitric oxide signalling.

The CB₂ mediated effects of THC increases the NO production via the induction of iNOS (Shmist *et al.,* 2006). Other cannabinoids such as CBD (Esposito *et al.,* 2006) and CBN (Esposito *et al.,* 2001) have been reported to inhibit inducible NO synthase (iNOS). Nitric oxide is synthesised from L-arginine by the three nitric oxide synthases: neuronal (nNOS), endothelial (eNOS) and (iNOS). The production of excess nitric oxide is associated with the initiation of apoptosis mediated through mitochondrial damage in breast cancer cells (Umansky *et al.,* 2000). Since NO is small and hydrophobic, it can easily diffuse through the mitochondrial membrane. The mitochondria are a major source of superoxide (O²⁻), which is continually formed by electron leakage from the respiratory chain. The major role of superoxide dismutases is to intercept O²⁻ and prevent the formation of peroxynitrite anion (ONOO⁻). When the concentration of NO increases, it competes with SODs, resulting in the formation of ONOO⁻. Cytoplasmic ONOO⁻ diffuses into the mitochondria, increasing the intramitochondrial ONOO⁻ concentration. ONOO⁻ further inactivates SOD, increasing the O²⁻ concentration in the mitochondria. This destructive cascade increases the concentration of ONOO⁻ which damages respiratory chain complexes I, II and III, and citric acid cycle enzymes, inhibits glycolysis through S-nitrosylating of glyceraldehyde 3-phosphate dehydrogenase (GAPDH), ultimately impairing cellular ATP synthesis. The depletion of ATP inhibits the plasma membrane and endoplasmic reticulum Ca²⁺ ATPases, which disrupts the calcium homeostasis in the cell. Excess NO causes oxidative stress, disruption of energy metabolism, calcium homeostasis and mitochondrial function. These disturbances subsequently lead to the induction of apoptosis (Murphy, 1999).

In contrast, nitric oxide has also been reported to induce cell proliferation (Villalobo, 2006). This paradoxical effect is linked to the NO concentration, where low concentrations induce cell proliferation, while higher concentrations induce cell cycle arrest and cell death (Villalobo, 2006). THC has been documented to increase iNOS expression, thereby increasing NO production, mediated via CB₂ (Shmist *et al.,* 2006). CBN reduces NO production via activation of the CB₁ receptor, decreasing the cAMP signalling cascade, which attenuates the activation of the transcription factor NF-κβ, necessary for the induction of the iNOS gene (Esposito *et al.,* 2001).

**B6** contained a higher THC concentration than **C6,** therefore it could be possible that THC produced a high enough NO concentration, which was not sufficiently counteracted by the lower CBN concentration, ultimately inducing cell cycle arrest and/or cell death in the MCF-10A cell line (Figure 14). **C6** contained a lower THC concentration, therefore less NO may have been produced by THC, which was effectively counteracted by the higher CBN concentration, possibly decreasing the NO concentration to a level that promotes rather than inhibits cell proliferation.

The cytotoxicity of **B6** and **C6** was further evaluated in white blood cells (Figure 14b). Mature, differentiated white blood cells originate from hemopoietic progenitor cells. Once committed, they differentiate into specific white blood cells e.g. neutrophils, basophils, eosinophils and monocytes. These committed progenitor cells divide a limited number of times before they terminally differentiate into mature white blood cells where after cell division ceases. As such, the positive control camptothecin, which inhibits cell proliferation, would not be an effective positive control in the screening of white blood cells. Curcumin was used as the positive control during the screening of **B6** and **C6** in the white blood cells. Curcumin displays a vast range of bioactivity and has repeatedly been reported to induce apoptosis in various cell types (Hu *et al.,* 2018, Mortezaee *et al.,* 2019).

The half-life of WBCs varies depending on the type of WBC. Neutrophils display the shortest half-life of approximately 7 hours, therefore, to prevent spontaneous cell death from interfering with the cytotoxic studies, treatment exposure was limited to 24 hours as opposed to the 48 hours of treatment exposure for other experiments in this study.

THC induces cell cycle arrest and apoptosis in various cell lines at different concentrations depending on the cell line, mediated via the CB₂ receptor. The CB₂ receptor is commonly expressed in immune cells, including white blood cells (WBC). Hence, it was expected that **B6** and **C6** would result in significant cell cycle arrest or cell death in the WBC mediated by THC. Both synergistic cannabinoid combinations, **B6** and **C6,** displayed limited growth inhibition in the WBC. WBC are terminally differentiated and do not proliferate. Therefore, the results normalised to the DMSO vehicle control, would essentially indicate no growth inhibition, as the cell number of the treated and untreated cells remain the same. This was supported by the lack of growth inhibition induced by camptothecin, a known agent to induce cell growth inhibition (Figure 14). Therefore, the limited growth inhibition observed in the WBC after treatment with **B6** and **C6** can be misinterpreted for displaying limited cytotoxicity when cell number is used to evaluate the cytotoxic effects of **B6** and **C6** against the WBC. Alternatively, a molecular approach should be taken, in which cell cycle markers such as cyclin D and cyclin B are quantified.

In conclusion, the screening of the individual cannabinoids displayed lower inhibitory concentrations in the MDA-MB-231 cells. At ICso, the order of cannabinoid potency remained the same against the MDA-MB-231 and MCF-7 cell line and was as follows: CBD>CBN>CBG>THC (Table 1). The similar trend in potency could be attributed to similar cannabinoid receptor expression levels across the two cell lines. The CB₁ expression levels were reported to be low across the two cell, while the CB₂ receptor expression levels were indiscernible in the MDA-MB-231 and MCF-7 cell lines (McKallip *et al.,* 2005). THC exerts its anti-proliferative effects via the CB₂ receptor (Caffarel *et al.,* 2006, Caffarel *et al.,* 2008). With low CB₂ expression levels, it could explain why THC displayed the highest IC values of the four cannabinoids tested.

CBD is known to exert its anti-proliferative effects in a cannabinoid-receptor independent mechanism (Sultan *et al.,* 2018, Kiskova *et al.,* 2019), thereby its anti-proliferative effects are not limited by the low cannabinoid expression levels, making CBD the most cytotoxic cannabinoid of the four. Additionally, at IC₇₅ and IC₉₀, there was a shift in the cannabinoid potency order: CBN>CBD>CBG>THC (Table 1). This could be attributed to the slope of the CBD dose response curve, thereby highlighting why synergistic analysis should incorporate the shape of the dose response curve.

When the two cannabinoid combinations were evaluated for potential synergistic interactions, the MDA-MB-231 cell line displayed antagonism for all the combinations tested, at IC₅₀, IC₇₅ and IC₉₀ (Table 3). The combinations evaluated against the MCF-7 cells resulted in a single, moderately synergistic combination between THC and CBD; however, this combination displayed different CI values at different effect levels (Table 3). At 90% inhibition, the interaction between THC and CBD was additive, while at 50% and 75%, the interaction was synergistic, despite the fact that the ratio between THC and CBD remained constant at the different effect levels. This highlighted the importance of how concentration of the cannabinoids in the combination affected the outcome of the interaction. Additionally, the three combinations with the lowest inhibitory concentrations in the MDA-MB-231 cells were: CBG:CBN, CBG:CBD and CBG:CBD (Table 2). All three these combinations excluded THC. The three most potent combinations in the MCF-7 (ER+) cells were: THC:CBN, THC:CBD and CBN:CBD (Table 2); two of the three combinations contained THC. THC has been reported to display enhanced anti-proliferative effects in ER+ cell lines through the modulation of oestrogen signalling (Takeda *et al.,* 2013).

The combinations of four cannabinoids only displayed synergistic interactions in the MCF-7 cell line (Table 4), with **A6, B6** and **C6** displaying synergism. These combinations contained the same concentration of CBD, with varying concentrations of THC in the order of high to low: **A6>B6>C6.** Although THC displayed enhanced anti-proliferative effects in the MCF-7 cells, several studies document the psychotic activity of THC (Koguel *et al.,* 2018, Lafaye *et al.,* 2017) and THC-induced DNA damage in neuronal cells (Kopjar *et al.,* 2019). Therefore, the two synergistic combinations containing the lowest THC content were selected for further evaluation.

The ideal outcome of a synergistic interaction is the reduction of the required dose, to decrease the overall cost of treatment, and minimised cytotoxicity to non-cancerous cells. **C6** and **B6** attained both these favourable features (Table 5 and Figure 14). Each of the four cannabinoids comprising the combinations, **B6** and **C6** displayed favourable dose reduction indices (DRI>1) ranging between 1.46 - 12.80 and 1.43 - 11.66, respectively (Table 5). Furthermore, screening of **B6** and **C6** against the non-cancerous cell line, MCF-10A, revealed significant cytotoxicity for **B6** (approximately 55%), while **C6** displayed limited cytotoxicity of only 4%. **B6** and **C6** comprised of the same CBD and CBG concentrations, 16 µM and 3 µM, respectively. The THC and CBN concentrations varied with **B6** consisted of THC:CBN at 17 µM: 4 µM, while **C6** consisted of THC:CBN at 13 µM:7 µM. It has been reported that the cannabinoid receptor expression levels decrease when non-cancerous cells are treated with a cannabinoid agonist, thereby displaying limited toxicity, when compared to cancer cells (Hermanson and Marnett, 2011). Therefore, the difference in cytotoxicity displayed by **B6** and **C6** in the MCF-10A screening results (Figure 14) could not be attributed to the increased cannabinoid receptor mediated effects of THC. Alternatively, this could be explained by NO signalling. At high NO production, cell cycle arrest or cell death is induced (Murphy, 1999), while at low NO concentrations, cell proliferation is enhanced (Villalobo, 2006). THC has been documented to increase NO production (Esposito *et al.,* 2006) while CBN has been documented to reduce NO production (Esposito *et al.,* 2001). With higher THC and lower CBN to counterbalance the NO produced, **B6** may have induced a higher NO concentration which favoured cell cycle arrest or cell death (Figure 14), while the lower THC and higher CBN concentration of **C6** produced less NO, thereby favouring cell proliferation (Figure 14). This once again highlights why the modulation of cannabinoid concentrations are of utmost importance.

Screening **B6** and **C6** against the white blood cells resulted in limited cytotoxicity (Figure 14), which contradicts the results obtained for screening of **B6** in the MCF-10A cells. This also contradicts literature since THC has been shown to induce cell cycle arrest in WBC (Nahtigal *et al.,* 2016, Zou and Kumar, 2018). When the WBC cells were treated with camptothecin, a known agent to induce cell cycle arrest, limited cytotoxicity was also observed (Figure 14). Since white blood cells cease proliferation after they terminally differentiate (Alberts, 2002), even if cell cycle arrest was induced, the cell number of the untreated and treated cells would remain comparable. Therefore, the anti-proliferative effects induced by **B6** and **C6** cannot be evaluated in cells that do not proliferate. Alternatively, the effects exerted by **B6** and **C6** should be evaluated on a molecular level to avoid the misinterpretation of limited cytotoxicity induced by **B6** and **C6** in white blood cells. In conclusion, synergistic cannabinoid combinations show promise as alternative cancer therapy, especially with their limited toxicity to the MCF-10A cell line, which is a representative cell line for non-cancerous cells.

### Mechanism of action

Cannabinoids have been documented to induce cell death via apoptosis, autophagy and paraptosis. The molecular mechanisms of paraptosis are not as well defined, when compared to the other cell death mechanisms, *e.g.* apoptosis and autophagy. In experiments conducted by the inventors, **C6** decreased the overall lysosome number but increased the lysosome size, associated with a starvation response which could be induced by autophagy or paraptosis. Both these mechanisms induce vacuoles, autophagy induces the formation of double membraned vacuoles, while paraptosis induces the formation of single membraned vacuoles. The only way to distinguish between these two types of vacuoles is to perform transmission electron microscopy to observe the ultrastructural morphology.

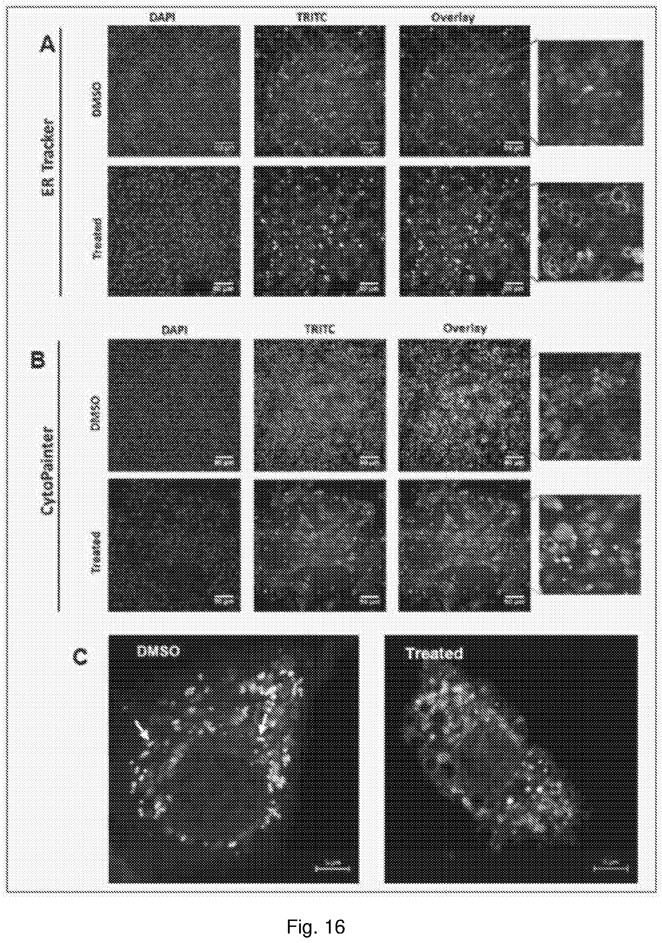

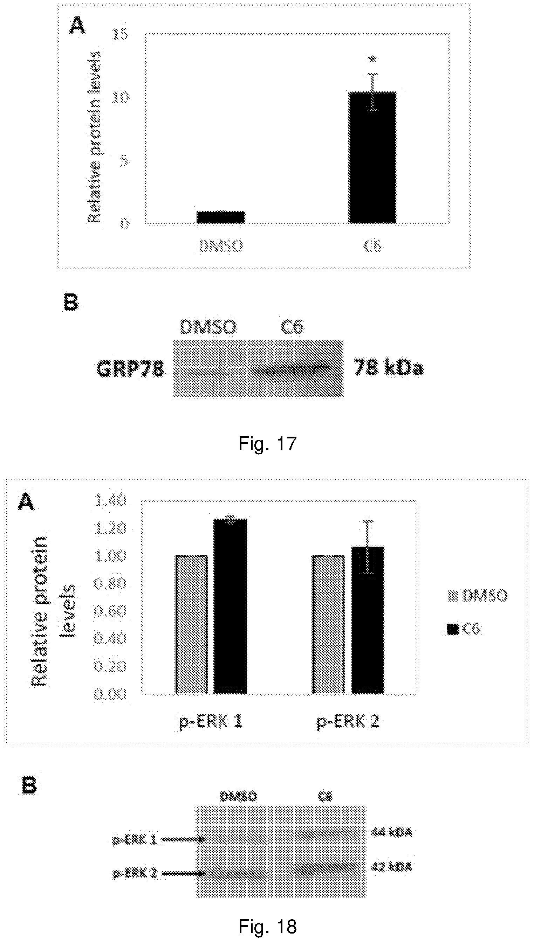

**C6** treated MCF-7 cells showed several markers of paraptosis, which included cytoplasmic vacuolation (Figure 15) dilation of the mitochondria (Figure 16), vacuole membrane derived from the endoplasmic reticulum (Figure 16a), ER stress (Figure 17), sustained p-ERK activity (Figure 18) and the retention of plasma membrane integrity (Figure 19). Paraptosis has been shown to disrupt the BK channels present on the mitochondria and ERM membrane, which increases the organelle Na⁺ concentration and water content, resulting in mitochondrial and ERM dilation as well as extensive cytoplasmic vacuolation (Figure 15). Mitochondrial staining showed that the mitochondrial structure was altered, indicating mitochondrial dilation (Figure 16c). Loss of mitochondrial structure disrupts the ability of the mitochondria to produce and maintain ATP levels. This in turn could initiate a starvation response, increasing lysosome size, which can be associated with both autophagy and paraptosis. Staining the cells with ER tracker^{™} showed that the vacuole membrane was derived from the ERM membrane (Figure 16a); however, due to the principal behind binding, dilation of the ER could not be observed. Literature indicates that the depletion in ATP levels could also induce endoplasmic reticulum stress. ATP is required for protein folding and protein misfoldinginduces the ERM stress response. Western blot analysis of **C6** treated cells, showed that ERM stress was induced with the GRP78 protein expression levels being significantly increased (Figure 17). ERK plays an important role in cell proliferation, *e.g.* decreased ERK activity would inhibit cell proliferation. Western blot analysis; however, eluded to the complexity of the signalling pathway, since sustained expression of p-ERK (Figure 18) was observed, even though cell cycle arrest occurred (Figure 20). Cell cycle arrest was induced in the G2 phase (Figure 20) prior to the induction of apoptosis (Figure 19). **C6** induced apoptosis in approximately 55% of the cell population (Figure 19). Phosphatidylserine translocation is not a common marker of paraptosis, but rather apoptosis; however, one study has reported the translocation of PS with the induction of paraptosis. Since **C6** consists of four cannabinoids, it could be possible that both mechanisms were initiated. To determine whether both apoptosis and paraptosis were induced, the MCF-7 cell line could be co-treated with **C6** and an apoptosis inhibitor, to determine whether the percentage apoptotic cells are altered. During staining with Annexin V and PI, only 4% PI positive (necrotic) cells were observed (Figure 19). Positive staining for PI indicated loss of plasma membrane integrity. The limited PI-positive staining is an indication that the plasma membrane integrity was retained, a common feature of paraptosis.

In summary, treatment of the MCF-7 cells induced mitochondrial dilation, cytoplasmic vacuoles with membranes derived from the endoplasmic reticulum, retention of plasma membrane integrity, sustained p-ERK activity, endoplasmic reticulum stress and PS translocation preceded by G2 arrest. Taken together, these results indicated that **C6** induced its effect in MCF-7 cells through biochemical markers representative of paraptosis induction.

## Claims

1. A combination comprising Δ⁹-tetrahydrocannabinol (THC), cannabigerol (CBG), cannabinol (CBN), and cannabidiol (CBD) in a ratio of 3.7 parts THC, 1.0 parts CBG, 1.9 parts CBN, and 4.8 parts CBD, or wherein the combination comprises 5.5 parts THC, 1.0 parts CBG, 1.3 parts CBN, and 4.8 parts CBD, for use in a method of treating cancer.

2. A combination for use according to claim 1, wherein the cannabinoids are present at a concentration of 28.89 µM THC, 7.90 µM CBG, 14.82 µM CBN, and 38.00 µM CBD.

3. A combination for use according to claim 1, wherein the cannabinoids are present at a concentration of 12.68 µM THC, 3.46 µM CBG, 6.50 µM CBN, and 16.68 µM CBD.

4. A combination for use according to any one of claims 1- 3, wherein the cancer is breast cancer.

5. A pharmaceutical composition comprising 3.7 parts Δ⁹-tetrahydrocannabinol (THC), 1.0 parts cannabigerol (CBG), 1.9 parts cannabinol (CBN), and 4.8 parts cannabidiol (CBD), or 5.5 parts THC, 1.0 parts CBG, 1.3 parts CBN, and 4.8 parts CBD, and one or more pharmaceutically acceptable excipients.

6. The pharmaceutical composition according to claim 5, for use in a method of treating cancer.

7. The pharmaceutical composition for use according to claim 6, wherein the cancer is breast cancer.

## Patentansprüche

1. Kombination, umfassend Δ⁹-Tetrahydrocannabinol (THC), Cannabigerol (CBG), Cannabinol (CBN) und Cannabidiol (CBD) in einem Verhältnis von 3,7 Teilen THC, 1,0 Teilen CBG, 1,9 Teilen CBN und 4,8 Teilen CBD, oder wobei die Kombination 5,5 Teile THC, 1,0 Teile CBG, 1,3 Teile CBN und 4,8 Teile CBD umfasst, zur Verwendung in einem Verfahren zum Behandeln von Krebs.

2. Kombination zur Verwendung nach Anspruch 1, wobei die Cannabinoide in einer Konzentration von 28,89 µM THC, 7,90 µM CBG, 14,82 µM CBN und 38,00 µM CBD vorliegen.

3. Kombination zur Verwendung nach Anspruch 1, wobei die Cannabinoide in einer Konzentration von 12,68 µM THC, 3,46 µM CBG, 6,50 µM CBN und 16,68 µM CBD vorliegen.

4. Kombination zur Verwendung nach einem der Ansprüche 1 bis 3, wobei der Krebs Brustkrebs ist.

5. Pharmazeutische Zusammensetzung, umfassend 3,7 Teile Δ⁹-Tetrahydrocannabinol (THC), 1,0 Teile Cannabigerol (CBG), 1,9 Teile Cannabinol (CBN) und 4,8 Teile Cannabidiol (CBD) oder 5,5 Teile THC, 1,0 Teile CBG, 1,3 Teile CBN und 4,8 Teile CBD und einen oder mehrere pharmazeutisch verträgliche Hilfsstoffe.

6. Pharmazeutische Zusammensetzung nach Anspruch 5 zur Verwendung in einem Verfahren zum Behandeln von Krebs.

7. Pharmazeutische Zusammensetzung zur Verwendung nach Anspruch 6, wobei der Krebs Brustkrebs ist.

## Revendications

1. Combinaison comprenant du Δ⁹-tétrahydrocannabinol (THC), du cannabigérol (CBG), du cannabinol (CBN) et du cannabidiol (CBD) dans un rapport de 3,7 parties de THC, 1,0 partie de CBG, 1,9 partie de CBN et 4,8 parties de CBD, ou dans laquelle la combinaison comprend 5,5 parties de THC, 1,0 partie de CBG, 1,3 partie de CBN et 4,8 parties de CBD, pour une utilisation dans une méthode de traitement du cancer.

2. Combinaison pour une utilisation selon la revendication 1, dans laquelle les cannabinoïdes sont présents à une concentration de 28,89 µM de THC, 7,90 µM de CBG, 14,82 µM de CBN, et 38,00 µM de CBD.

3. Combinaison pour une utilisation selon la revendication 1, dans laquelle les cannabinoïdes sont présents à une concentration de 12,68 µM de THC, 3,46 µM de CBG, 6,50 µM de CBN, et 16,68 µM de CBD.

4. Combinaison pour une utilisation selon l'une quelconque des revendications 1 à 3, dans laquelle le cancer est un cancer du sein.

5. Composition pharmaceutique comprenant 3,7 parties de Δ⁹-tétrahydrocannabinol (THC), 1,0 partie de cannabigérol (CBG), 1,9 partie de cannabinol (CBN) et 4,8 parties de cannabidiol (CBD), ou 5,5 parties de THC, 1,0 partie de CBG, 1,3 partie de CBN et 4,8 parties de CBD, et un ou plusieurs excipients pharmaceutiquement acceptables.

6. Composition pharmaceutique selon la revendication 5, pour une utilisation dans une méthode de traitement du cancer.

7. Composition pharmaceutique pour une utilisation selon la revendication 6, dans laquelle le cancer est un cancer du sein.
